# EUROPEAN PATENT SPECIFICATION

(11) **EP 1 732 614 B1**
(45) Date of publication and mention of the grant of the patent: **24.12.2008**
(21) Application number: 05732726.4
(22) Date of filing: 07.04.2005
(51) Int. Cl.: A61K 48/00

(54) **COMPOSITIONS FOR TREATING NEUROPATHIC AND NEURODEGENERATIVE CONDITIONS**
ZUSAMMENSETZUNGEN ZUR BEHANDLUNG VON NEUROPATHISCHEN UND NEURODEGENERATIVEN ERKRANKUNGEN
COMPOSITIONS PERMETTANT DE TRAITER LES TROUBLES NEUROPATHIQUES ET NEURODEGENERATIFS

(30) Priority: 08.04.2004 US 560566 P; 29.11.2004 US 631455 P
(43) Date of publication of application: 20.12.2006
(73) Proprietor: Sangamo BioSciences, Inc., Richmond, CA 94804 (US)
(72) Inventor: MARTIN, J., Tyler, Fremont, California 94539 (US)
(74) Representative: Weiss, Wolfgang
(86) International application number: PCT/US2005/011838
(87) International publication number: WO 2005/099771

(56) References cited:
- WO-A-20/04053130
- US-A1- 2002 160 940
- US-A1- 2003 044 404
- LIU P-Q ET AL: "REGULATION OF AN ENDOGENOUS LOCUS USING A PANEL OF DESIGNED ZINC FINGER PROTEINS TARGETED TO ACCESSIBLE CHROMATIN REGIONS ACTIVATION OF VASCULAR ENDOTHELIAL GROWTH FACTOR A" JOURNAL OF BIOLOGICAL CHEMISTRY, AMERICAN SOCIETY OF BIOCHEMISTRY AND MOLECULAR BIOLOGY, US, vol. 276, no. 14, 6 April 2001 (2001-04-06), pages 11323-11334, XP000999288 ISSN: 0021-9258

## Description

### BACKGROUND

Diabetic neuropathies are a family of nerve disorders caused by diabetes. People with diabetes can, over time, experience damage to nerves throughout the body. Neuropathies lead to numbness and sometimes pain and weakness in the hands, arms, feet, and legs. These neurologic problems may also occur in every organ system, including the digestive tract, heart, and sex organs. People with diabetes can develop nerve problems at any time, but the longer a person has diabetes, the greater the risk.

The most common type of diabetic neuropathy is peripheral neuropathy, also called distal symmetric neuropathy, which affects the extremities (*e.g.,* arms, hands, legs, feet). In addition to peripheral neuropathy, diabetic neuropathies can occur within autonomic nerve systems, proximal (pain in the thighs, hips, or buttocks leading to weakness in the leg), and focal. Autonomic neuropathy may cause changes in digestion, bowel and bladder function, sexual response, and perspiration. It can also affect the nerves that serve the heart and control blood pressure. Autonomic diabetic neuropathy can also cause hypoglycemia (low blood sugar) unawareness, a condition in which people no longer experience the warning signs of hypoglycemia.

Although over half of diabetes patients experience some form of neuropathy, there are currently no treatments for neuropathy other than controlling the diabetic condition per se. It has been known for some time that diabetic patients do not convert the essential fatty acid (EFA) linoleic acid to gamma-linoleic acid (GLA), due to a deficit in the production of enzyme delta-6-desaturase and/or the enzyme delta 5-desaturase. *See, e.g.,* Keen et al. (1993) Diabetes Care. 16(1):8-15. Accordingly, some treatments of diabetic neuropathies have focused on encouraging nerve growth via changing the patient's nutritional intake.

Many other diseases result from neuropathy or neural degeneration, *i.e*., the death of neurons or the failure of damaged neurons to regenerate. These include, for example, amyotrophic lateral sclerosis (ALS, also known as Lou Gehrig's disease), which results from degeneration of motor neurons. Moreover, trauma to neural tissue, such as nerve crush and spinal cord injuries, can result in neuropathy; in which case treatments that stimulate neural regeneration would be advantageous.

Recently, several groups have reported that administration of neurotrophic molecules *per se* may help ameliorate nerve degeneration characteristic of diabetic neuropathy. For example, Schratzberger et al. J. Clin. Inv. (2001) 107, 1083-1092, demonstrated that gene transfer of vascular endothelial growth factor (VEGF) could reverse diabetic neuropathy characterized by a loss of axons and demyelination in the rat experimental model. *See, also*, Isner et al. (2001) Hum Gene Ther. 10;12(12):1593-4; Sondell et al. (2000) European J. Neurosciences 12:4243-4254; Sondell (1999) J. Neurosciences 19(14):5731-5740. In addition, anecdotal neurological improvements have been reported in patients with diabetes in a phase I/II dose-escalating trial of VEGF₁₆₅ gene therapy for critical limb ischemia. Simovic et al. Arch Neurol. (2001) 58:761-788.

However, modulation of the expression of neurotrophic proteins involved in nerve growth so as to treat neuropathies and neurodegenerative conditions, diabetic and otherwise, has not been previously described. The ability to stimulate nerve growth in a cell or group of cells, using one or more exogenous molecules, would have utility in treating and/or preventing all types of neuropathies and neurodegeneration, and in relieving the pain associated with certain of these conditions.

### SUMMARY

A variety of zinc finger proteins (ZFPs) and methods utilizing such proteins are provided for use in treating neuropathies. These include engineered zinc finger proteins, i.e., non-naturally occurring proteins which bind to a predetermined nucleic acid target sequence that are obtained, for example, by rational design or by selection from a library comprising a plurality of zinc finger proteins of different sequence. Such libraries can be either polypeptide libraries or libraries of polynucleotides which encode a plurality of zinc finger polypeptides of different sequence from which the plurality of proteins can be expressed.

The ZFPs can be placed in operative linkage with a regulatory domain (or functional domain) as part of a fusion protein. By selecting either an activation domain or repression domain for fusion with the ZFP, such fusion proteins can be used either to activate or to repress gene expression. Thus, by appropriate choice of the regulatory domain fused to the ZFP, one can selectively modulate the expression of a gene and hence modulate various physiological processes correlated with one or more genes. In the case of certain neuropathies, for example, by attaching an activation domain to a ZFP that binds to a target sequence within a gene encoding a neurotrophic product, and introducing such a fusion protein (or a nucleic acid encoding such a fusion protein) into a cell or tissue, one can enhance certain beneficial aspects associated with the neurotrophic properties of the target gene(s). In contrast, for neuropathies that are associated with over-expression of a gene, one can reduce expression of the gene by using ZFPs that are fused to a repression domain.

By selecting certain ZFPs, it is possible to tailor the extent to which a physiological process (*e.g*., nerve growth and/or function) can be modulated and tailor treatment. This can be achieved because multiple target sites (*e.g*., 9, 12, 15 or 18 base pair target sequences) in any given gene can be acted upon by the ZFPs provided herein and because a single ZFP can bind to a target site located in a plurality of genes. Thus, in some methods, a plurality of ZFPs are administered, which can then bind to different target sites located within the same gene. Such ZFPs can in some instances have a synergistic effect. In certain methods, the plurality of fusion proteins include different regulatory domains. In contrast, with some of the ZFPs provided herein, administration of a single ZFP can modulate expression of multiple genes because each gene includes the target site (*e.g*., within a region of sequence conservation among the different genes).

Also provided herein are polynucleotides and nucleic acids that encode the ZFPs disclosed herein. Additionally, pharmaceutical compositions containing the nucleic acids and/or ZFPs are also provided. For example, certain compositions include a nucleic acid comprising a sequence that encodes one of the ZFPs described herein operably linked to a regulatory sequence, combined with a pharmaceutically acceptable carrier or diluent, wherein the regulatory sequence allows for expression of the nucleic acid in a cell. Protein based compositions include a ZFP as disclosed herein and a pharmaceutically acceptable carrier or diluent.

Also provided are methods for preventing neural degeneration, as well as methods for stimulating neural regeneration, using the compositions disclosed herein

### BRIEF DESCRIPTION OF THE DRAWINGS

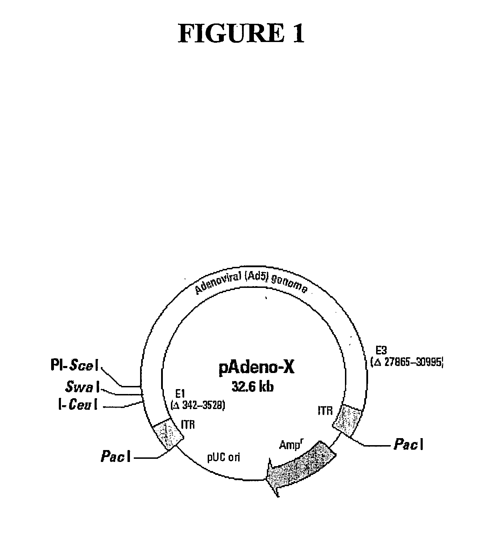

**Figure 1** shows a schematic diagram of an adenoviral vector.

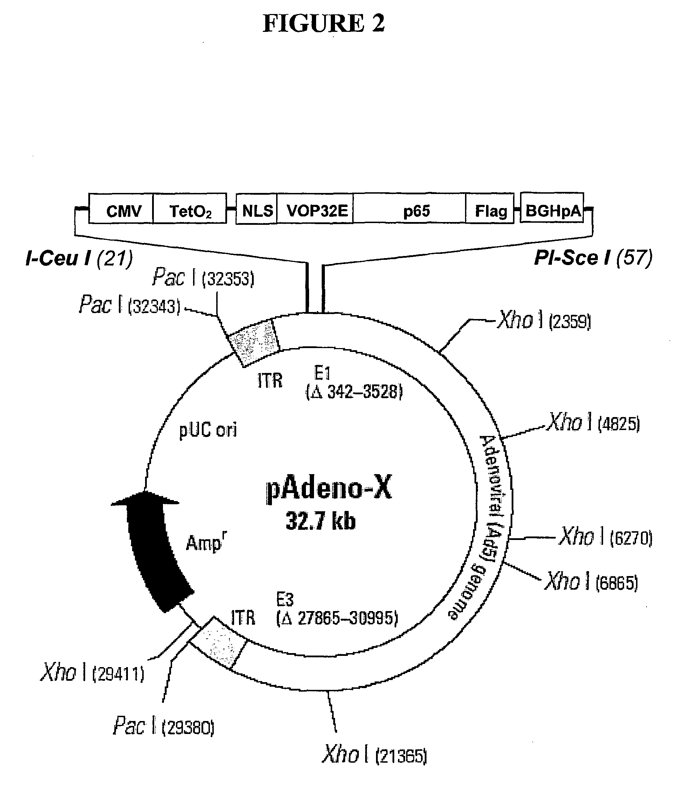

**Figure 2** shows a schematic diagram on an adenoviral vector encoding the NLS-VOP32E-p65-Flag fusion protein under the transcriptional control of a tetracycline-inducible CMV promoter and a bovine growth hormone polyadenylation signal. See Example 4 for details.

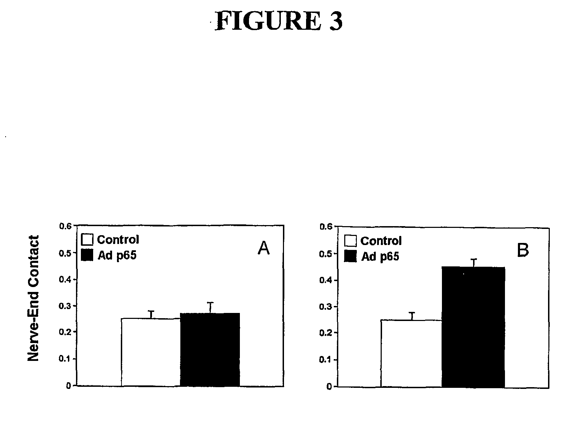

**Figures 3** **A and B** show percentage nerve endplate contact, determined by histological identification of motor endplates and nerve fibers, in cyosections of laryngeal muscle from rats in which the recurrent laryngeal nerve had been crushed, then treated with AdVOP32Ep65 (dark bars) or a control adenoviral vector (light bars). Measurements were made at 3 days after treatment (A) and at 7 days after treatment (B).

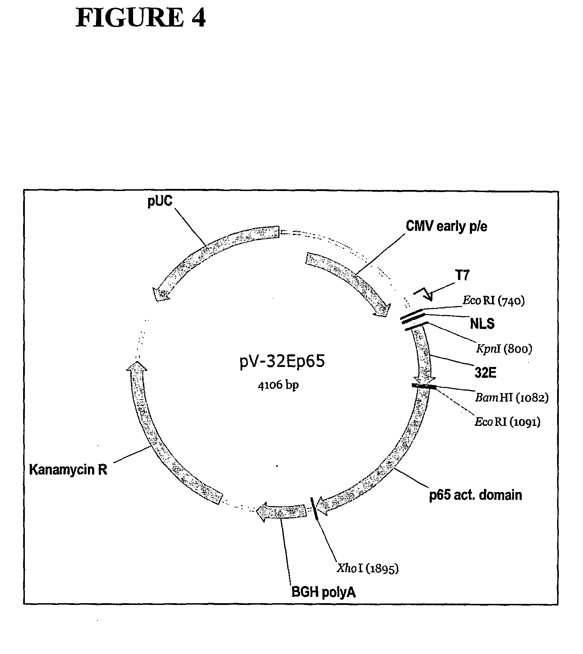

**Figure 4** is a schematic diagram of plasmid pV-32Ep65 which encodes a VEGF-activating zinc finger fusion protein. Abbreviations are as follows. pUC: vector backbone; Kanamycin R: kanamycin resistance gene; CMV early p/e: human cytomegalovirus early promoter/enhancer sequences; T7: bacteriophage T7 promoter; NLS: nuclear localization sequence; 32E: VOP32E zinc finger binding domain (Table 1); p65 act. domain: transcriptional activation domain from p65; BGH polyA: polyadenylation signal from bovine growth hormone gene. Recognition sites for restriction enzymes *Eco*RI, *Kpn*I, *Bam*HI and *Xho*I are also shown.

### DETAILED DESCRIPTION

Practice of the methods, as well as preparation and use of the compositions disclosed herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. *See,* for example, Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, "Chromatin" (P.M. Wassarman and A. P. Wolffe, eds.), Academic Press, San Diego, 1999; and METHODS IN MOLECULAR BIOLOGY, Vol. 119, "Chromatin Protocols" (P.B. Becker, ed.) Humana Press, Totowa, 1999.

### I. DEFINITIONS

The term "zinc finger protein" or "ZFP" refers to a protein having DNA binding domains that are stabilized by zinc. The individual DNA binding domains are typically referred to as "fingers." A ZFP has least one finger, typically two, three, four, five, six or more fingers. Each finger binds from two to four base pairs of DNA, typically three or four base pairs of DNA. A ZFP binds to a nucleic acid sequence called a target site or target segment. Each finger typically comprises an approximately 30 amino acid, zinc-chelating, DNA-binding subdomain. An exemplary motif characterizing one class of these proteins (C₂H₂ class) is -Cys-(X)₂₋₄-Cys-(X)₁₂-His-(X)₃₋₅-His (where X is any amino acid) (SEQ ID NO:147). Additional classes of zinc finger proteins are known and are useful in the practice of the methods, and in the manufacture and use of the compositions disclosed herein (see, e.g., Rhodes et al. (1993) Scientific American 268:56-65 and US Patent Application Publication No. 2003/0108880). Studies have demonstrated that a single zinc finger of this class consists of an alpha helix containing the two invariant histidine residues coordinated with zinc along with the two cysteine residues of a single beta turn (see, e.g., Berg & Shi, Science 271:1081-1085 (1996)).

A "target site" is the nucleic acid sequence recognized by a ZFP. A single target site typically has about four to about ten base pairs. Typically, a two-fingered ZFP recognizes a four to seven base pair target site, a three-fingered ZFP recognizes a six to ten base pair target site, a four-finger ZFP recognizes a 12-14 bp target sequence and a six-fingered ZFP recognizes an 18-21 bp target sequence, which can comprise two adjacent nine to ten base pair target sites or three adjacent 6-7 bp target sites.

A "target subsite" or "subsite" is the portion of a DNA target site that is bound by a single zinc finger, excluding cross-strand interactions. Thus, in the absence of cross-strand interactions, a subsite is generally three nucleotides in length. In cases in which a cross-strand interaction occurs (i.e., a "D-able subsite," see co-owned WO 00/42219) a subsite is four nucleotides in length and overlaps with another 3- or 4-nucleotide subsite.

"Kd" refers to the dissociation constant for a binding molecule, i.e., the concentration of a compound (e.g., a zinc finger protein) that gives half maximal binding of the compound to its target (i.e., half of the compound molecules are bound to the target) under given conditions (i.e., when [target]<<Kd), as measured using a given assay system (see, e.g., U.S. Pat. No. 5,789,538). The assay system used to measure the Kd should be chosen so that it gives the most accurate measure of the actual Kd of the ZFP. Any assay system can be used, as long is it gives an accurate measurement of the actual Kd of the ZFP. In one embodiment, the Kd for a ZFP is measured using an electrophoretic mobility shift assay ("EMSA"). Unless an adjustment is made for ZFP purity or activity, the Kd calculations may result in an overestimate of the true Kd of a given ZFP. Preferably, the Kd of a ZFP used to modulate transcription of a gene is less than about 100 nM, more preferably less than about 75 nM, more preferably less than about 50 nM, most preferably less than about 25 nM.

A "gene," for the purposes of the present disclosure, includes a DNA region encoding a gene product, as well as all DNA regions that regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions. A wide variety of genes can be targeted to treat diabetic neuropathy using the ZFPs and methods described herein, including various growth factors, enzymes, etc. For example, VEGF has been shown to have neurotrophic effects. VEGF genes are defined and described in detail in U.S. Patent Application No. 20030021776A1, incorporated by reference in its entirety herein.

The term "gene" includes nucleic acids that are substantially identical to a native gene. The terms "identical" or percent "identity," in the context of two or more nucleic acids or polypeptides, refer to two or more sequences or subsequences that are the same or have a specified percentage of nucleotides or amino acid residues that are the same, when compared and aligned for maximum correspondence, as measured using a sequence comparison algorithm such as those described below for example, or by visual inspection.

The phrase "substantially identical," in the context of two nucleic acids or polypeptides, refers to two or more sequences or subsequences that have at least 75%, preferably at least 85%, more preferably at least 90%, 95% or higher or any integral value therebetween nucleotide or amino acid residue identity, when compared and aligned for maximum correspondence, as measured using a sequence comparison algorithm such as those described below for example, or by visual inspection. Preferably, the substantial identity exists over a region of the sequences that is at least about 10, preferably about 20, more preferable about 40-60 residues in length or any integral value therebetween, preferably over a longer region than 60-80 residues, more preferably at least about 90-100 residues, and most preferably the sequences are substantially identical over the full length of the sequences being compared, such as the coding region of a nucleotide sequence for example.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection [see generally, Current Protocols in Molecular Biology, (Ausubel, F. M. et al., eds.) John Wiley & Sons, Inc., New York (1987-1999, including supplements such as supplement 46 (April 1999)]. Use of these programs to conduct sequence comparisons are typically conducted using the default parameters specific for each program.

Another example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. This is referred to as the neighborhood word score threshold (Altschul et al, supra.). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. For determining sequence similarity the default parameters of the BLAST programs are suitable. The BLASTN program (for nucleotide sequences) uses as defaults a word length (W) of 11, an expectation (E) of 10, M=5, N=-4, and a comparison of both strands. For amino acid sequences, the BLASTP program uses as defaults a word length (W) of 3, an expectation (E) of 10, and the BLOSUM62 scoring matrix. The TBLATN program (using protein sequence for nucleotide sequence) uses as defaults a word length (W) of 3, an expectation (E) of 10, and a BLOSUM 62 scoring matrix. (see Henikoff & Henikoff, Proc. Natl. Acad. Sci. USA 89:10915 (1989)). 11171 In addition to calculating percent sequence identity, the BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

Another indication that two nucleic acid sequences are substantially identical is that the two molecules hybridize to each other under stringent conditions. "Hybridizes substantially" refers to complementary hybridization between a probe nucleic acid and a target nucleic acid and embraces minor mismatches that can be accommodated by reducing the stringency of the hybridization media to achieve the desired detection of the target polynucleotide sequence. The phrase "hybridizing specifically to", refers to the binding, duplexing, or hybridizing of a molecule only to a particular nucleotide sequence under stringent conditions when that sequence is present in a complex mixture (e.g., total cellular) DNA or RNA.

A further indication that two nucleic acid sequences or polypeptides are substantially identical is that the polypeptide encoded by the first nucleic acid is immunologically cross reactive with the polypeptide encoded by the second nucleic acid, as described below.

"Conservatively modified variations" of a particular polynucleotide sequence refers to those polynucleotides that encode identical or essentially identical amino acid sequences, or where the polynucleotide does not encode an amino acid sequence, to essentially identical sequences. Because of the degeneracy of the genetic code, a large number of functionally identical nucleic acids encode any given polypeptide. For instance, the codons CGU, CGC, CGA, CGG, AGA, and AGG all encode the amino acid arginine. Thus, at every position where an arginine is specified by a codon, the codon can be altered to any of the corresponding codons described without altering the encoded polypeptide. Such nucleic acid variations are "silent variations," which are one species of "conservatively modified variations." Every polynucleotide sequence described herein that encodes a polypeptide also describes every possible silent variation, except where otherwise noted. One of skill will recognize that each codon in a nucleic acid (except AUG, which is ordinarily the only codon for methionine) can be modified to yield a functionally identical molecule by standard techniques. Accordingly, each "silent variation" of a nucleic acid that encodes a polypeptide is implicit in each described sequence.

A polypeptide is typically substantially identical to a second polypeptide, for example, where the two peptides differ only by conservative substitutions. A "conservative substitution," when describing a protein, refers to a change in the amino acid composition of the protein that does not substantially alter the protein's activity. Thus, "conservatively modified variations" of a particular amino acid sequence refers to amino acid substitutions of those amino acids that are not critical for protein activity or substitution of amino acids with other amino acids having similar properties (e.g., acidic, basic, positively or negatively charged, polar or non-polar, etc.) such that the substitutions of even critical amino acids do not substantially alter activity. Conservative substitution tables providing functionally similar amino acids are well known in the art. See, e.g., Creighton (1984) Proteins, W. H. Freeman and Company. In addition, individual substitutions, deletions or additions which alter, add or delete a single amino acid or a small percentage of amino acids in an encoded sequence are also "conservatively modified variations."

A "functional fragment" or "functional equivalent" of a protein, polypeptide or nucleic acid is a protein, polypeptide or nucleic acid whose sequence is not identical to the full-length protein, polypeptide or nucleic acid, yet retains the same function as the full-length protein, polypeptide or nucleic acid. A functional fragment can possess more, fewer, or the same number of residues as the corresponding native molecule, and/or can contain one ore more amino acid or nucleotide substitutions. Methods for determining the function of a nucleic acid (e.g., coding function, ability to hybridize to another nucleic acid, binding to a regulatory molecule) are well known in the art. Similarly, methods for determining protein function are well known. For example, the DNA-binding function of a polypeptide can be determined, for example, by filter-binding, electrophoretic mobility-shift, or immunoprecipitation assays. See Ausubel et al., supra. The ability of a protein to interact with another protein can be determined, for example, by co-immunoprecipitation, two-hybrid assays or complementation, both genetic and biochemical. See, for example, Fields et al. (1989) Nature 340:245-246; U.S. Pat. No. 5,585,245 and PCT WO 98/44350.

The terms "nucleic acid," "polynucleotide," and "oligonucleotide" are used interchangeably and refer to a deoxyribonucleotide or ribonucleotide polymer in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties. In general, an analogue of a particular nucleotide has the same base-pairing specificity; i.e., an analogue of A will base-pair with T. Thus, the term polynucleotide sequence is the alphabetical representation of a polynucleotide molecule. This alphabetical representation can be input into databases in a computer having a central processing unit and used for bioinformatics applications such as functional genomics and homology searching. The terms additionally encompass nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, and peptide-nucleic acids (PNAs). The nucleotide sequences are displayed herein in the conventional 5'-3' orientation.

"Chromatin" is the nucleoprotein structure comprising the cellular genome. "Cellular chromatin" comprises nucleic acid, primarily DNA, and protein, including histones and non-histone chromosomal proteins. The majority of eukaryotic cellular chromatin exists in the form of nucleosomes, wherein a nucleosome core comprises approximately 150 base pairs of DNA associated with an octamer comprising two each of histones H2A, H2B, H3 and H4; and linker DNA (of variable length depending on the organism) extends between nucleosome cores. A molecule of histone HI is generally associated with the linker DNA. For the purposes of the present disclosure, the term "chromatin" is meant to encompass all types of cellular nucleoprotein, both prokaryotic and eukaryotic. Cellular chromatin includes both chromosomal and episomal chromatin.

A "chromosome" is a chromatin complex comprising all or a portion of the genome of a cell. The genome of a cell is often characterized by its karyotype, which is the collection of all the chromosomes that comprise the genome of the cell. The genome of a cell can comprise one or more chromosomes.

An "episome" is a replicating nucleic acid, nucleoprotein complex or other structure comprising a nucleic acid that is not part of the chromosomal karyotype of a cell. Examples of episomes include plasmids and certain viral genomes.

An "exogenous molecule" is a molecule that is not normally present in a cell, but can be introduced into a cell by one or more genetic, biochemical or other methods. Normal presence in the cell is determined with respect to the particular developmental stage and environmental conditions of the cell. Thus, for example, a molecule that is present only during embryonic development of muscle is an exogenous molecule with respect to an adult muscle cell. An exogenous molecule can comprise, for example, a functioning version of a malfunctioning endogenous molecule or a malfunctioning version of a normally functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded; can be linear, branched or circular; and can be of any length. Nucleic acids include those capable of forming duplexes, as well as triplex-forming nucleic acids. See, for example, U.S. Pat. Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

An exogenous molecule can be the same type of molecule as an endogenous molecule, e.g., protein or nucleic acid (i.e., an exogenous gene), providing it has a sequence that is different from an endogenous molecule. Methods for the introduction of exogenous molecules into cells are known to those of skill in the art and include, but are not limited to, lipid-mediated transfer (i.e., liposomes, including neutral and cationic lipids), electroporation, direct injection, cell fusion, particle bombardment, calcium phosphate co-precipitation, DEAE-dextran-mediated transfer and viral vector-mediated transfer.

By contrast, an "endogenous molecule" is one that is normally present in a particular cell at a particular developmental stage under particular environmental conditions.

An "endogenous gene" is a gene that is present in its normal genomic and chromatin context. An endogenous gene can be present, e.g., in a chromosome, an episome, a bacterial genome or a viral genome.

The phrase "adjacent to a transcription initiation site" refers to a target site that is within about 50 bases either upstream or downstream of a transcription initiation site. "Upstream" of a transcription initiation site refers to a target site that is more than about 50 bases 5' of the transcription initiation site (i.e., in the non-transcribed region of the gene). "Downstream" of a transcription initiation site refers to a target site that is more than about 50 bases 3' of the transcription initiation site.

A "fusion molecule" is a molecule in which two or more subunit molecules are linked, typically covalently. The subunit molecules can be the same chemical type of molecule, or can be different chemical types of molecules. Examples of the first type of fusion molecule include, but are not limited to, fusion polypeptides (for example, a fusion between a ZFP DNA-binding domain and a transcriptional activation domain) and fusion nucleic acids (for example, a nucleic acid encoding the fusion polypeptide described supra). Examples of the second type of fusion molecule include, but are not limited to, a fusion between a triplex-forming nucleic acid and a polypeptide, and a fusion between a minor groove binder and a nucleic acid.

"Gene expression" refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include RNAs that are modified, by processes such as capping, polyadenylation, methylation, and editing, and proteins modified by, for example, methylation, acetylation, phosphorylation, ubiquitination, ADP-ribosylation, myristilation, and glycosylation.

"Gene activation" refers to any process that results in an increase in production of a gene product. A gene product can be either RNA (including, but not limited to, mRNA, rRNA, tRNA, and structural RNA) or protein. Accordingly, gene activation includes those processes that increase transcription of a gene and/or translation of a mRNA. Examples of gene activation processes that increase transcription include, but are not limited to, those that facilitate formation of a transcription initiation complex, those that increase transcription initiation rate, those that increase transcription elongation rate, those that increase processivity of transcription and those that relieve transcriptional repression (by, for example, blocking the binding of a transcriptional repressor). Gene activation can constitute, for example, inhibition of repression as well as stimulation of expression above an existing level. Examples of gene activation processes that increase translation include those that increase translational initiation, those that increase translational elongation and those that increase mRNA stability. In general, gene activation comprises any detectable increase in the production of a gene product, in some instances an increase in production of a gene product by about 2-fold, in other instances from about 2- to about 5-fold or any integer therebetween, in still other instances between about 5- and about 10-fold or any integer therebetween, in yet other instances between about 10- and about 20-fold or any integer therebetween, sometimes between about 20- and about 50-fold or any integer therebetween, in other instances between about 50- and about 100-fold or any integer therebetween, and in yet other instances between 100-fold or more.

"Gene repression" and "inhibition of gene expression" refer to any process that results in a decrease in production of a gene product. A gene product can be either RNA (including, but not limited to, mRNA, rRNA, tRNA, and structural RNA) or protein. Accordingly, gene repression includes those processes that decrease transcription of a gene and/or translation of a mRNA. Examples of gene repression processes which decrease transcription include, but are not limited to, those which inhibit formation of a transcription initiation complex, those which decrease transcription initiation rate, those which decrease transcription elongation rate, those which decrease processivity of transcription and those which antagonize transcriptional activation (by, for example, blocking the binding of a transcriptional activator). Gene repression can constitute, for example, prevention of activation as well as inhibition of expression below an existing level. Examples of gene repression processes that decrease translation include those that decrease translational initiation, those that decrease translational elongation and those that decrease mRNA stability. Transcriptional repression includes both reversible and irreversible inactivation of gene transcription. In general, gene repression comprises any detectable decrease in the production of a gene product, in some instances a decrease in production of a gene product by about 2-fold, in other instances from about 2- to about 5-fold or any integer therebetween, in yet other instances between about 5- and about 10-fold or any integer therebetween, in still other instances between about 10- and about 20-fold or any integer therebetween, sometimes between about 20- and about 50-fold or any integer therebetween, in other instances between about 50- and about 100-fold or any integer therebetween, in still other instances 100-fold or more. In yet other instances, gene repression results in complete inhibition of gene expression, such that no gene product is detectable.

"Modulation" refers to a change in the level or magnitude of an activity or process. The change can be either an increase or a decrease. For example, modulation of gene expression includes both gene activation and gene repression. Modulation can be assayed by determining any parameter that is indirectly or directly affected by the expression of the target gene. Such parameters include, e.g., changes in RNA or protein levels, changes in protein activity, changes in product levels, changes in downstream gene expression, changes in reporter gene transcription (luciferase, CAT, β-galactosidase, β-glucuronidase, green fluorescent protein (see, e.g., Mistili & Spector, Nature Biotechnology 15:961-964 (1997)); changes in signal transduction, phosphorylation and dephosphorylation, receptor-ligand interactions, second messenger concentrations (e.g., cGMP, cAMP, IP3, and Ca2+), cell growth, and vascularization. These assays can be in vitro, in vivo, and ex vivo. Such functional effects can be measured by any means known to those skilled in the art, e.g., measurement of RNA or protein levels, measurement of RNA stability, identification of downstream or reporter gene expression, e.g., via chemiluminescence, fluorescence, colorimetric reactions, antibody binding, inducible markers, ligand binding assays; changes in intracellular second messengers such as cGMP and inositol triphosphate (IP3); changes in intracellular calcium levels; cytokine release, and the like.

A "regulatory domain" or "functional domain" refers to a protein or a protein domain that has transcriptional modulation activity when tethered to a DNA binding domain, i.e., a ZFP. Typically, a regulatory domain is covalently or non-covalently linked to a ZFP (e.g., to form a fusion molecule) to effect transcription modulation. Regulatory domains can be activation domains or repression domains. Activation domains include, but are not limited to, VP16, VP64 and the p65 subunit of nuclear factor Kappa-B. Repression domains include, but are not limited to, KOX, KRAB MBD2B and v-ErbA. Additional regulatory domains include, e.g., transcription factors and co-factors (e.g., MAD, ERD, SID, early growth response factor 1, and nuclear hormone receptors), endonucleases, integrases, recombinases, methyltransferases, histone acetyltransferases, histone deacetylases etc. Activators and repressors include co-activators and co-repressors (see, e.g., Utley et al., Nature 394:498-502 (1998)). Alternatively, a ZFP can act alone, without a regulatory domain, to effect transcription modulation.

The term "operably linked" or "operatively linked" is used with reference to a juxtaposition of two or more components (such as sequence elements), in which the components are arranged such that both components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. An operatively linked transcriptional regulatory sequence is generally joined in cis with a coding sequence, but need not be directly adjacent to it. For example, an enhancer can constitute a transcriptional regulatory sequence that is operatively linked to a coding sequence, even though they are not contiguous.

With respect to fusion polypeptides, the term "operably linked" or "operatively linked" can refer to the fact that each of the components performs the same function in linkage to the other component as it would if it were not so linked. For example, with respect to a fusion polypeptide in which a ZFP DNA-binding domain is fused to a transcriptional activation domain (or functional fragment thereof), the ZFP DNA-binding domain and the transcriptional activation domain (or functional fragment thereof) are in operative linkage if, in the fusion polypeptide, the ZFP DNA-binding domain portion is able to bind its target site and/or its binding site, while the transcriptional activation domain (or functional fragment thereof) is able to activate transcription.

The term "recombinant," when used with reference to a cell, indicates that the cell replicates an exogenous nucleic acid, or expresses a peptide or protein encoded by an exogenous nucleic acid. Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques.

A "recombinant expression cassette," "expression cassette" or "expression construct" is a nucleic acid construct, generated recombinantly or synthetically, that has control elements that are capable of effecting expression of a structural gene that is operatively linked to the control elements in hosts compatible with such sequences. Expression cassettes include at least promoters and optionally, transcription termination signals. Typically, the recombinant expression cassette includes at least a nucleic acid to be transcribed (e.g., a nucleic acid encoding a desired polypeptide) and a promoter. Additional factors necessary or helpful in effecting expression can also be used as described herein. For example, an expression cassette can also include nucleotide sequences that encode a signal sequence that directs secretion of an expressed protein from the host cell. Transcription termination signals, enhancers, and other nucleic acid sequences that influence gene expression, can also be included in an expression cassette.

A "promoter" is defined as an array of nucleic acid control sequences that direct transcription. As used herein, a promoter typically includes necessary nucleic acid sequences near the start site of transcription, such as, in the case of certain RNA polymerase II type promoters, a TATA element, CCAAT box, SP-1 site, etc. As used herein, a promoter also optionally includes distal enhancer or repressor elements, which can be located as much as several thousand base pairs from the start site of transcription. The promoters often have an element that is responsive to transactivation by a DNA-binding moiety such as a polypeptide, e.g., a nuclear receptor, Gal4, the lac repressor and the like.

A "constitutive" promoter is a promoter that is active under most environmental and developmental conditions. An "inducible" promoter is a promoter that is active under certain environmental or developmental conditions.

A "weak promoter" refers to a promoter having about the same activity as a wild type herpes simplex virus ("HSV") thymidine kinase ("tk") promoter or a mutated HSV tk promoter, as described in Eisenberg & McKnight, Mol. Cell. Biol. 5:1940-1947 (1985).

An "expression vector" is a nucleic acid construct, generated recombinantly or synthetically, with a series of specified nucleic acid elements that permit transcription of a particular nucleic acid in a host cell, and optionally integration or replication of the expression vector in a host cell. The expression vector can be part of a plasmid, virus, or nucleic acid fragment, of viral or non-viral origin. Typically, the expression vector includes an "expression cassette," which comprises a nucleic acid to be transcribed operably linked to a promoter. The term expression vector also encompasses naked DNA operably linked to a promoter.

By "host cell" is meant a cell that contains an expression vector or nucleic acid, either of which optionally encodes a ZFP or a ZFP fusion protein. The host cell typically supports the replication or expression of the expression vector. Host cells can be prokaryotic cells such as, for example, E. coli, or eukaryotic cells such as yeast, fungal, protozoal, higher plant, insect, or amphibian cells, or mammalian cells such as CHO, HeLa, 293, COS-1, and the like, e.g., cultured cells (in vitro), explants and primary cultures (in vitro and ex vivo), and cells in vivo.

The term "naturally occurring," as applied to an object, means that the object can be found in nature, as distinct from being artificially produced by humans.

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins. The polypeptide sequences are displayed herein in the conventional N-terminal to C-terminal orientation.

A "subsequence" or "segment" when used in reference to a nucleic acid or polypeptide refers to a sequence of nucleotides or amino acids that comprise a part of a longer sequence of nucleotides or amino acids (e.g., a polypeptide), respectively.

"Neuropathy" refers to a clinical condition characterized death of neurons and/or glial cells, or by failure of neuron regeneration after nerve damage. "Diabetic neuropathy" broadly refers to progressive nerve damage associated with diabetes. Neuropathies include peripheral neuropathies, autonomic neuropathies and focal neuropathies.

The terms "treating" and "treatment" as used herein refer to reduction in severity and/or frequency of symptoms, elimination of symptoms and/or underlying cause, prevention of the occurrence of symptoms and/or their underlying cause, and improvement or remediation of damage.

By an "effective" amount (or "therapeutically effective" amount) of a pharmaceutical composition is meant a nontoxic amount of the agent that is sufficient to provide the desired effect. The term refers to an amount sufficient to treat a subject. Thus, the term therapeutic amount refers to an amount sufficient to remedy a disease state or symptoms, by preventing, hindering, retarding or reversing the progression of the disease or any other undesirable symptoms whatsoever. The term prophylactically effective amount refers to an amount given to a subject that does not yet have the disease, and thus is an amount effective to prevent, hinder or retard the onset of a disease.

### II. OVERVIEW

A variety of methods are provided herein for treating neuropathies and neurodegenerative conditions. In particular, the methods described herein involve modulation of the expression of genes whose products are involved in various neuropathic or neurodegenerative conditions, (*e.g*., diabetic neuropathy, ALS). Such genes include, but are not limited to, those encoding neurotrophic growth factors such as, for example, vascular endothelial growth factor (VEGF), nerve growth factor (NGF), insulin-like growth factor 2 (IGF-2) and the like, as well as other genes involved in metabolism, for example those encoding products involved in fatty acid metabolism to produce gamma-linoleic acid (GLA) such as the genes encoding the enzymes delta-6-desaturase and delta 5-desaturase.

In some instances, such methods involve contacting a cell or population of cells such as in an organism, with one or more zinc finger proteins (ZFPs) that bind to specific sequences in one or more of the genes described above (*e.g*., VEGF, IGF-2). In certain methods, one ZFP is administered and is able to bind to a target site in different genes (e.g., a target site in VEGF-A, VEGF-B and VEGF-C or a target site in VEGF-A and IGF-2). Other methods involve administering a plurality of different ZFPs that bind to multiple target sites within a particular gene. Alternatively, a cell or cell population can be contacted with one or more polynucleotides encoding a ZFP, such that the polynucleotide enters one or more cells, the encoded ZFP is expressed, and the protein binds to its target sequence, thereby modulating expression of the gene (or genes) in which the target sequence is located.

Thus, also provided herein are a variety of zinc finger proteins (and/or nucleic acids encoding such zinc finger proteins) that are engineered to specifically recognize and bind to particular nucleic acid segments (target sites or target sequences), in one or more genes involved in neuropathic conditions (e.g., VEGF, IGF-2), modulate the expression of that gene (or those genes) and thereby treat neuropathies. Treatment of neuropathy includes both prevention of neural degeneration and stimulation of neural regeneration.

In one embodiment, the ZFPs are linked to regulatory domains to create chimeric transcription factors to activate or repress transcription of target genes.

With certain ZFPs, expression of genes can be enhanced; with certain other ZFPs, expression can be repressed. In general, the target sites to which the ZFPs bind are sites from which binding results in activation or repression of expression of a targeted gene (*e.g*., VEGF). The target site can be adjacent to, upstream of, and/or downstream of the transcription start site (defined as nucleotide +1). As indicated above, some of the present ZFPs modulate the expression of a single gene. Other ZFPs modulate the expression of a plurality of genes. Thus, depending upon the particular ZFP(s) utilized, one can tailor the level at which one or more genes are expressed. In addition, multiple ZFPs or ZFP fusion molecules, having distinct target sites, can be used to regulate a single gene.

By virtue of the ability of the ZFPs to bind to target sites and influence expression of selected genes, the ZFPs provided herein can be used to treat a wide range of neuropathies, both by prevention of nerve degeneration and by stimulation of nerve regeneration. In certain applications, the ZFPs can be used to activate expression of certain genes to trigger beneficial nerve growth in cell populations, both in vitro and in vivo. Such activation can be utilized for example to promote the formation of nerve tissue and, accordingly, as treatment for neuropathies.

Other methods involve repression of genes that are either over-expressed in neuropathies, or whose expression contributes to a pathology associated with a neuropathy. For example, in diabetics, excess glucose is converted to sorbitol by aldose reductase (AR). Cells are non-permeable to sorbitol; consequently, there is a tendency for sorbitol to accumulate within cells in diabetics. Repression of AR expression is therefore expected to reduce the severity of certain diabetic symptoms which result from accumulation of sorbitol. Accordingly, a ZFP engineered to bind a target site in the AR gene, also comprising a transcriptional repression domain, can be used for treatment.

### III. ZINC FINGER PROTEINS FOR REGULATING GENE EXPRESSION

### A. GENERAL

The zinc finger proteins (ZFPs) disclosed herein are proteins that can bind to DNA in a sequence-specific manner. As indicated above, these ZFPs can be used to treat a number of neuropathic conditions. An exemplary motif characterizing one class of these proteins, the C₂H₂ class, is -Cys-(X)₂₋₄-Cys-(X)₁₂-His-(X)₃₋₅-His (where X is any amino acid) (SEQ. ID. NO:1). Several structural studies have demonstrated that the zinc finger domain contains an alpha helix containing the two invariant histidine residues and a beta turn containing the two invariant cysteine residues, wherein the two invariant histidine residues and the two invariant cysteine residues are coordinated through a zinc ion. However, the ZFPs provided herein are not limited to this particular class. Additional classes of zinc finger proteins are known and can also be used in the methods and compositions disclosed herein (see, e.g., Rhodes, et al. (1993) Scientific American 268:56-65 and US Patent Application Publication No. 2003/0108880). In certain ZFPs, a single zinc finger domain is about 30 amino acids in length. Zinc finger domains are involved not only in DNA-recognition, but also in RNA binding and in protein-protein binding.

The x-ray crystal structure of Zif268, a three-finger domain from a murine transcription factor, has been solved in complex with a cognate DNA-sequence and shows that each finger can be superimposed on the next by a periodic rotation. The structure suggests that each finger interacts independently with DNA over 3 base-pair intervals, with side-chains at positions -1, 2, 3 and 6 on each recognition helix making contacts with nucleotides in their respective DNA triplet subsites. The amino terminus of Zif268 is situated at the 3' end of the DNA strand with which it makes most contacts. Some zinc fingers can bind to a fourth base in a target segment. If the strand with which a zinc finger protein makes most contacts is designated the target strand, some zinc finger proteins bind to a three base triplet in the target strand and a fourth base on the nontarget strand. The fourth base is complementary to the base immediately 3' of the three base subsite.

### B. EXEMPLARY ZFPS

ZFPs that have been engineered to bind to target sites in the sequence of a gene involved in a neuropathic condition are disclosed herein. Engineering of ZFPs can be accomplished, for example, by rational design or through empirical selection from randomized libraries. See for example, co-owned US Patents 6,453,242 and 6,534,261. Non-limiting examples of genes encoding products that may be involved in neuropathies include neurotrophic growth factors such as VEGF, IGF-2, as well as enzymes (*e.g*., enzymes involved in GLA synthesis). Thus, the ZFPs can include a variety of different component fingers of varying amino acid composition, provided the ZFP binds to a target site in the gene or genes of interest.

The target sites can be located upstream or downstream of the transcriptional start site (defined as nucleotide +1). Some of the target sites include 9 nucleotides, some can include 12 nucleotides, whereas other sites include 18 nucleotides. One feature of these target sites is that binding of a ZFP, or a fusion protein including a ZFP and one or more regulatory domains, to the target site can affect the level of expression of one or more genes. Exemplary VEGF genes that can be regulated by the ZFPs provided herein include, but are not limited to, VEGF-A (including isoforms VEGF-A121, VEGF-A145, VEGF-A165, VEGF-A189, and VEGF-A206), VEGF B (including isoforms VEGF-B 167, and VEGF-B 186), VEGF C, VEGF D, the viral VEGF-like proteins (viral VEGF-E) and mammalian VEGF-E, VEGF-H, VEGF-R, VEGF-X, VEGF-138 and P1GF (including P1GF-1 and P1GF-2). *See, also*, U.S. Patent Application No. 20030021776A1.

The target sites can be located adjacent to the transcription start site or can be located significantly upstream or downstream of the transcription start site. Some target sites are located within a single gene such that binding of a ZFP to the target affects the expression of a single gene. Other target sites are located within multiple genes such that the binding of a single ZFP can modulate the expression of multiple genes. In still other instances multiple ZFPs can be used, each recognizing targets in the same gene or in different genes.

The ZFPs that bind to these target sites typically include at least one zinc finger but can include a plurality of zinc fingers (e.g., 2, 3, 4, 5, 6 or more fingers). Usually, the ZFPs include at least three fingers. Certain of the ZFPs include four or six fingers. The ZFPs that include three fingers typically recognize a target site that includes 9 or 10 nucleotides; ZFPs that include four fingers typically recognize a target site that includes 12 to 14 nucleotides; while ZFPs having six fingers can recognize target sites that include 18 to 21 nucleotides. The ZFPs can also be fusion proteins that include one or more regulatory domains, which domains can be transcriptional activation or repression domains.

Accordingly, specific examples of such ZFPs are disclosed in Tables 1 and 2. The VEGF sequences examined for target sites include the sequences for VEGF-A (see GenBank accession number AF095785), VEGF-B (see GenBank accession number U80601--from -0.4 kb to +0.32 kb), VEGF-C (see GenBank accession number AF020393) and VEGF-D genes (see, HSU69570 and HSY12864), as well as the sequences for P1GF (see, GenBank accession number AC015837) and viral VEGF-E genes (see, GenBank accession number AF106020 and Meyer, M., et al. (1999) EMBO J. 18:363-74; GenBank accession number S67520 and Lyttle, D. J. et al. (1994) J. Virol. 68:84-92; and GenBank accession number AF091434). Thus, for example, the nucleotide sequence of the VEGF-A gene examined for target sites extended from 2.3 kb upstream of the transcriptional start site to 1.1 kb downstream of the transcriptional start site.

In certain embodiments, diabetic neuropathy(ies) and other neuropathic conditions (*e.g*. nerve trauma, spinal cord injury) are treated using one or more ZFPs that bind to a target site in a VEGF gene. The location(s) of the target site(s) for the exemplary ZFPs disclosed in Tables 1 and 2 in the various VEGF genes is(are) summarized in Table 3. The first column in this table is an internal reference name for a ZFP and corresponds to the same name in column 1 of Tables 1 and 2. The location of the 5' end of the target site in various VEGF gene sequences is listed in the remaining columns. Negative numbers in Table 3 refer to the number of nucleotides upstream of the transcriptional start site (defined as nucleotide +1), whereas positive numbers indicate the number of nucleotides downstream of the transcriptional start site.

Thus, as indicated herein, certain ZFPs described herein can be utilized to treat diabetic neuropathies by modulating the activity of single genes, while other ZFPs can be utilized to regulate expression of a plurality of genes. By judicious selection of the various ZFPs provided herein and/or combinations thereof, one can tailor which genes are modulated and, accordingly, which neuropathy(ies) is(are) treated.

**TABLE 1 Target sites and recognition region sequences of human VEGF-targeted ZFPs**

| ZFP NAME | TARGET | SEQ. ID NO | F 1 | SEQ ID NO | F 2 | SEQ ID NO | F 3 | SEQ ID NO | K_{d} (nM) |
|---|---|---|---|---|---|---|---|---|---|
| BVO 13A | ATGGACGGG | 1 | RSDHLAR | 30 | DRSNLTR | 59 | RSDALTQ | 88 | <.02 |
| EP10A | KGGGGCTGG | 2 | RSDHLTT | 31 | DRSHLAR | 60 | RSDHLSK | 89 | 0.35 |
| GATA82Z678 | GAGKGKGYG | 3 | RLDSLLR | 32 | DRDHLTR | 61 | RSDNLAR | 90 | 1.8 |
| HBV 3 | GGGGGAGGW | 4 | QTGHLRR | 33 | QSGHLQR | 62 | RSDHLSR | 91 | 30 |
| HP38 4A | GGDTGGGGG | 5 | RSDHLAR | 34 | RSDHLTT | 63 | QRAHLAR | 92 | 0.75 |
| HUM 17A | ARGGGGGAG | 6 | RSDNLAR | 35 | RSDHLSR | 64 | RSDNLTQ | 93 | <.02 |
| HUM 19A | TGGGCAGAC | 7 | DRSNLTR | 36 | QSGDLTR | 65 | RSDHLTT | 94 | 0.02 |
| MTS 5A | TGGGGGTGG | 8 | RSDHLTT | 37 | RSDHLTR | 66 | RSDHLTT | 95 | 0.07 |
| MX1E | ATGGACGGG | 9 | RSDHLAR | 38 | DRSNLTR | 67 | RSDALSA | 96 | 3.4 |
| PDF 5A | GYAGGGGCC | 10 | DRSSLTR | 39 | RSDHLSR | 68 | QSGSLTR | 97 | .23 |
| RAT 24A | GDGGAAGHC | 11 | ERGTLAR | 40 | QSGNLAR | 69 | RSDALAR | 98 | <.02 |
| SAN 16A | AKGGAAGGG | 12 | RSDHLAR | 41 | QSGNLAR | 70 | RSDALRQ | 99 | 1.03 |
| USX 3A | GCCGGGGAG | 13 | RSDNLTR | 42 | RSDHLTR | 71 | DRSDLTR | 100 | 0.06 |
| VEGF 1 | GGGGAGGVK | 14 | TTSNLRR | 43 | RSSNLQR | 72 | RSDHLSR | 101 | 2.83 |
| VEGF 1* | GGGGAGGVK | 15 | TTSNLRR | 44 | RSSNLQR | 73 | RSDHLSR | 102 | 3 |
| VEGF 1A | GGGGAGGVK | 16 | TTSNLRR | 45 | RSDNLQR | 74 | RSDHLSR | 103 | 0.2 |
| VEGF 1B | GGGGAGGAT | 17 | QSSNLAR | 46 | RSDNLQR | 75 | RSDHLSR | 104 | 2 |
| VEGF 1C | GGGGVGGAT | 18 | TTSNLAR | 47 | RSDNLQR | 76 | RSDHLSR | 105 | 1 |
| VEGF 1D | GGGGAGGMT | 19 | QSSNLRR | 48 | RSDNLQR | 77 | RSDHLSR | 106 | 2 |
| VG 10A | GAWGGGGGC | 20 | DSGHLTR | 49 | RSDHLTR | 78 | QSGNLTR | 107 | ND |
| VG 1B | ATGGGGGTG | 21 | RSDALTR | 50 | RSDHLTR | 79 | RSDALTQ | 108 | ND |
| VG 4A | GGGGGCTGG | 22 | RSDHLTT | 51 | DRSHLAR | 80 | RSDHLSR | 109 | ND |
| VG 8A | GDGTGGGGN | 23 | QSSHLAR | 52 | RSDHLTT | 81 | RSDALAR | 110 | .35 |
| VOP 28A-2 | GGGGGCGCT | 24 | QSSDLRR | 53 | DRSHLAR | 82 | RSDHLSR | 111 | <.02 |
| VOP 30A-4 | GCTGGGGGC | 25 | DRSHLTR | 54 | RSDHLTR | 83 | QSSDLTR | 112 | <.02 |
| VOP 32A-6 | GGGGGTGAC | 26 | DRSNLTR | 55 | MSHHLSR | 84 | RSDHLSR | 113 | <.02 |
| VOP 32B-7 | GGGGGTGAC | 27 | DRSNLTR | 56 | TSGHLVR | 85 | RSDHLSR | 114 | <.02 |
| VOP 35A-10 | GCTGGAGCA | 28 | QSGSLTR | 57 | QSGHLQR | 86 | QSSDLTR | 115 | <.02 |
| ZEN-7A 1 | GGGGGHGCT | 29 | QSSDLRR | 58 | QSSHLAR | 87 | RSDHLSR | 116 | .63 |
| VOP 29A-3 | GAGGCTTGG | 138 | RSDHLTT | 51 | QSSDLTR | 112 | RSDNLTR | 42 | <.02 |
| VOP 32-C | GGGGGTGAC | 26 | DRSNLTR | 55 | TSGHLTR | 139 | RSDHLSR | 68 | ND |
| VOP 32-D | GGGGGTGAC | 26 | DRSNLTR | 55 | TSGHLIR | 140 | RSDHLSR | 68 | ND |
| VOP 32-E | GGGGGTGAC | 26 | DRSNLTR | 55 | TSGHLSR | 141 | RSDHLSR | 68 | ND |
| VOP 32-F | GGGGGTGAC | 26 | DRSNLTR | 55 | TSGHLAR | 142 | RSDHLSR | 68 | ND |
| VOP 32-G | GGGGGTGAC | 26 | DRSNLTR | 55 | TSGHLRR | 143 | RSDHLSR | 68 | ND |
| VOP 32-H | GGGGGTGAC | 26 | DRSNLTR | 55 | TAGHLVR | 144 | RSDHLSR | 68 | ND |
| VOP 32-I | GGGGGTGAC | 26 | DRSNLTR | 55 | TTGHLVR | 145 | RSDHLSR | 68 | ND |
| VOP 32-J | GGGGGTGAC | 26 | DRSNLTR | 55 | TKDHLVR | 146 | RSDHLSR | 68 | ND |

**TABLE 2 Target sites and recognition region sequences of human VEGF-targeted ZFPs**

| ZFP NAME | TARGET | SEQ ID NO | F 1 | SEQ ID NO | F 2 | SEQ ID NO | F 3 | SEQ ID NO | F 4 | SEQ ID NO | F 5 | SEQ ID NO | F 6 | SEQ ID NO |
|---|---|---|---|---|---|---|---|---|---|---|---|---|---|---|
| BVO 10A-9A | GTGGAGGGGGTCGGGGCT | 117 | QSSDLRR | 120 | RSDHLTR | 123 | DRSALAR | 126 | RSDHLAR | 129 | RSDNLAR | 132 | RSDALTR | 135 |
| BVO 12A-11B | GGAGAGGGGGCYGCAGTG | 118 | RSDALTR | 121 | QSGDLTR | 124 | ERGDLTR | 127 | RSDHLAR | 130 | RSDNLAR | 133 | QSGHLQR | 136 |
| BVO 14B-13A | ATGGACGGGtGAGGYGGYG | 119 | RSDELTR | 122 | RSDELTR | 125 | RSDNLAR | 128 | RSDHLAR | 131 | DRSNLTR | 134 | RSDALTQ | 137 |

**TABLE 3 Locations of Target Site in VEGF Sequences**

| ZFP NAME | VEGF A | VEGF B | VEGF C | VEGF D | VEGF E (P1DGF) | Viral VEGF |
|---|---|---|---|---|---|---|
| BVO 13A | +851 | | | | | |
| EP10A | -1083 | -31 | -252 | | | |
| | +534 | | | | | |
| GATA82Z7678 | -485 | -170 | | | +183 | |
| HBV 3 | +779 | | -245 | | | |
| HP38 4A | -2248 | -119 | +479 | +805 | -29 | |
| | -1413 | | +510 | | +210 | |
| | -1055 | | | | | |
| | -633 | | | | | |
| HUM 17A | -1002 | | | | -33 | |
| | +472 | | | | | |
| HUM 19A | -1016 | | | | | |
| MTS 5A | -2251 | | | | +213 | |
| MX1E | +851 | | | | | |
| PDF 5A | +590 | | | | -748 | |
| RAT 24A | +711 | | | | | |
| SAN 16A | -1954 | | | | | |
| USX 3A | +554 | | -230 | | | |
| | +928 | | | | | |
| VEGF 1 | -8 | | | | -454 | |
| | | | | | -348 | |
| | | | | | -36 | |
| VEGF 1*3 | -8 | | | | -454 | |
| | | | | | -348 | |
| | | | | | -36 | |
| VEGF 1A | -8 | | | | -454 | |
| | | | | | -348 | |
| | | | | | -36 | |
| VEGF 1B | -8 | | | | | |
| VEGF 1C | -8 | | | | | |
| VEGF 1D | -8 | | | | | |
| VG 10A | -1412 | -774 | | | | |
| | -354 | | | | | |
| VG 1B | -2252 | -943 | | | | |
| VG 4A | -1083 | -31 | -252 | | | |
| VG 8A | -2248 | -119 | +479 | +313 | -903 | +575 |
| | -633 | -784 | +510 | +805 | -29 | |
| | -475 | | | | -22 | |
| | -391 | | | | +179 | |
| | | | | | +210 | |
| VOP 28A-2 | -573 | | +61 | | | |
| VOP 30A-4 | +42 | | -481 | | | |
| | +530 | | | | | |
| VOP 32A-6 | +434 | | | | | |
| VOP 32B-7 | +434 | | | | | |
| VOP 35A-10 | +892 | | | | | |
| ZEN-7A 1 | -1273 | -945 | +61 | | -675 | |
| | -573 | | | | | |
| BVO 10A-9A | +621 | | | | | |
| BVO 12A-11B | +806 | | | | | |
| BVO 14B-13A | +851 | | | | | |
| VOP 29A-3 | +5 | | | | | |
| VOP 32C | +434 | | | | | |
| VOP 32D | +434 | | | | | |
| VOP 32E | +434 | | | | | |
| VOP 32F | +434 | | | | | |
| VOP 32G | +434 | | | | | |
| VOP 32H | +434 | | | | | |
| VOP 32I | +434 | | | | | |
| VOP 32J | +434 | | | | | |

### IV. CHARACTERISTICS OF ZFPS

Zinc finger proteins are formed from zinc finger components. For example, zinc finger proteins can have one to thirty-seven fingers, commonly having 2, 3, 4, 5 or 6 fingers. A zinc finger protein recognizes and binds to a target site (sometimes referred to as a target segment) that represents a relatively small subsequence within a target gene. Each component finger of a zinc finger protein can bind to a subsite within the target site. The subsite includes a triplet of three contiguous bases all on the same strand (sometimes referred to as the target strand). The subsite may or may not also include a fourth base on the opposite strand that is the complement of the base immediately 3' of the three contiguous bases on the target strand. In many zinc finger proteins, a zinc finger binds to its triplet subsite substantially independently of other fingers in the same zinc finger protein. Accordingly, the binding specificity of zinc finger protein containing multiple fingers is usually approximately the aggregate of the specificities of its component fingers. For example, if a zinc finger protein is formed from first, second and third fingers that individually bind to triplets XXX, YYY, and ZZZ, the binding specificity of the zinc finger protein is 3'XXX YYY ZZZ5'.

The relative order of fingers in a zinc finger protein from N-terminal to C-terminal determines the relative order of triplets in the 3' to 5' direction in the target. For example, if a zinc finger protein comprises from N-terminal to C-terminal first, second and third fingers that individually bind, respectively, to triplets 5' GAC3', 5'GTA3' and 5" GGC3' then the zinc finger protein binds to the target segment 3'CAGATGCGG5' (SEQ ID NO:148). If the zinc finger protein comprises the fingers in another order, for example, second finger, first finger, third finger, then the zinc finger protein binds to a target segment comprising a different permutation of triplets, in this example, 3'ATGCAGCGG5' (SEQ ID NO:149). See Berg & Shi, Science 271, 1081-1086 (1996). The assessment of binding properties of a zinc finger protein as the aggregate of its component fingers may, in some cases, be influenced by context-dependent interactions of multiple fingers binding in the same protein.

Two or more zinc finger proteins can be linked to have a target specificity that is the aggregate of that of the component zinc finger proteins (see e.g., Kim & Pabo, Proc. Natl. Acad. Sci. U.S.A. 95, 2812-2817 (1998)). For example, a first zinc finger protein having first, second and third component fingers that respectively bind to XXX, YYY and ZZZ can be linked to a second zinc finger protein having first, second and third component fingers with binding specificities, AAA, BBB and CCC. The binding specificity of the combined first and second proteins is thus 3'XXXYYYZZZ_AAABBBCCC5', where the underline indicates a short intervening region (typically 0-5 bases of any type). In this situation, the target site can be viewed as comprising two target segments separated by an intervening segment.

Linkage can be accomplished using any of the following peptide linkers:
T G E K P: (SEQ ID NO: 150) (Liu et al., 1997, supra.); (G₄S)n (SEQ ID NO:151) (Kim et al., Proc. Natl. Acad. Sci. U.S.A. 93:1156-1160 (1996.); GGRRGGGS; (SEQ ID NO:152) LRQRDGERP; (SEQ ID NO:153) LRQKDGGGSERP; (SEQ ID NO:154) LRQKD(G₃S)₂ERP (SEQ ID NO:155).

Alternatively, flexible linkers can be rationally designed using computer programs capable of modeling both DNA-binding sites and the peptides themselves or by phage display methods. In a further variation, noncovalent linkage can be achieved by fusing two zinc finger proteins with domains promoting heterodimer formation of the two zinc finger proteins. For example, one zinc finger protein can be fused with fos and the other with jun (see Barbas et al., WO 95/119431).

Linkage of two or more zinc finger proteins is advantageous for conferring a unique binding specificity within a mammalian genome. A typical mammalian diploid genome consists of 3 x 10e9 bp. Assuming that the four nucleotides A, C, G, and T are randomly distributed, a given 9 bp sequence is present approximately 23,000 times. Thus a ZFP recognizing a 9 bp target with absolute specificity would have the potential to bind to.about.23,000 sites within the genome. An 18 bp sequence is present about once in a random DNA sequence whose complexity is ten times that of a mammalian genome.

A component finger of zinc finger protein typically contains about 30 amino acids and, in one embodiment, has the following motif (N-C):
Cys-(X)₂₋₄-Cys-X.X.X.X.X.X.X.X.X.X.X.X-His-(X)₃₋₅-His (SEQ ID NO:156)

The two invariant histidine residues and two invariant cysteine residues in a single beta turn are coordinated through zinc atom (see, e.g., Berg & Shi, Science 271, 1081-1085 (1996)). The above motif shows a numbering convention that is standard in the field for the region of a zinc finger conferring binding specificity (the "recognition region). The amino acid on the left (N-terminal side) of the first invariant His residue is assigned the number +6, and other amino acids further to the left are assigned successively decreasing numbers. The alpha helix begins at residue 1 and extends to the residue following the second conserved histidine. The entire helix is therefore of variable length, between 11 and 13 residues.

### V. DESIGN OF ZFPs

The ZFPs provided herein are engineered to recognize a selected target site in a gene associated with one or more diabetic neuropathies (e.g., one or more VEGF genes).

The process of designing or selecting a ZFP typically starts with a natural ZFP as a source of framework residues. The process of design or selection serves to define nonconserved positions (i.e., positions -1 to +6) so as to confer a desired binding specificity. One suitable ZFP is the DNA binding domain of the mouse transcription factor Zif268. The DNA binding domain of this protein has the amino acid sequence:
YACPVESCDRRFSRSDELTRHIRIHTGQKP (F1) (SEQ ID NO:157)
FQCRICMRNFSRSDHLTTHIRTHTGEKP (F2) (SEQ ID NO:158)
FACDICGRKFARSDERKRHTKIHLRQK (F3) SEQ ID NO:159)
and binds to a target 5' GCG TGG GCG 3' (SEQ ID NO:160).

Another suitable natural zinc finger protein as a source of framework residues is Sp-1. The Sp-1 sequence used for construction of zinc finger proteins corresponds to amino acids 531 to 624 in the Sp-1 transcription factor. This sequence is 94 amino acids in length. *See, e.g.,* U.S. Patent Application No. 20030021776 for the sequence of Sp1 and an alternate form of Sp-1, referred to as an Sp-1 consensus sequence.

Sp-1 binds to a target site 5'GGG GCG GGG3' (SEQ ID NO:161).

There are a number of substitution rules that assist rational design of some zinc finger proteins. For example, ZFP DNA-binding domains can be designed and/or selected to recognize a particular target site as described in co-owned US Patents 6,453,242 and 6,534,261 and US Patent Application Publication 2003/0068675; as well as U.S. Pat. Nos. 5,789,538; 6,007,408; 6,013,453; 6,140,081; and 6,140,466; and PCT publications WO 95/19431, WO 98/53057; WO 98/53058, WO 98/53059; WO 98/53060; WO 98/54311, WO 00/23464 and WO 00/27878. In one embodiment, a target site for a zinc finger DNA-binding domain is identified according to site selection rules disclosed in co-owned US Patent 6,453,242. In a certain embodiments, a ZFP can selected as described in co-owned WO 02/077227. See also WO 96/06166; Desjarlais & Berg, PNAS 90, 2256-2260 (1993); Choo & Klug, PNAS 91, 11163-11167 (1994); Desjarlais & Berg, PNAS 89, 7345-7349 (1992); and Jamieson et al., Biochemistry 33:5689-5695 (1994).

Many of these rules are supported by site-directed mutagenesis of the three-finger domain of the ubiquitous transcription factor, Sp-1 (Desjarlais and Berg, 1992; 1993). One of these rules is that a 5' G in a DNA triplet can be bound by a zinc finger incorporating arginine at position 6 of the recognition helix. Another substitution rule is that a G in the middle of a subsite can be recognized by including a histidine residue at position 3 of a zinc finger. A further substitution rule is that asparagine can be incorporated to recognize A in the middle of a triplet, aspartic acid, glutamic acid, serine or threonine can be incorporated to recognize C in the middle of a triplet, and amino acids with small side chains such as alanine can be incorporated to recognize T in the middle of a triplet. A further substitution rule is that the 3' base of a triplet subsite can be recognized by incorporating the following amino acids at position -1 of the recognition helix: arginine to recognize G, glutamine to recognize A, glutamic acid (or aspartic acid) to recognize C, and threonine to recognize T. Although these substitution rules are useful in designing zinc finger proteins they do not take into account all possible target sites. Furthermore, the assumption underlying the rules, namely that a particular amino acid in a zinc finger is responsible for binding to a particular base in a subsite is only approximate. Context-dependent interactions between proximate amino acids in a finger or binding of multiple amino acids to a single base or vice versa can cause variation of the binding specificities predicted by the existing substitution rules. Accordingly, in certain embodiments, a ZFP DNA-binding domain of predetermined specificity is obtained according to the methods described in co-owned WO 02/077227 and/or US Patent Application Publication 2003/0068675.

Any suitable method known in the art can be used to design and construct nucleic acids encoding ZFPs, e.g., phage display, random mutagenesis, combinatorial libraries, computer/rational design, affinity selection, PCR, cloning from cDNA or genomic libraries, synthetic construction and the like. (see, e.g., U.S. Pat. No. 5,786,538; Wu et al., PNAS 92:344-348 (1995); Jamieson et al., Biochemistry 33:5689-5695 (1994); Rebar & Pabo, Science 263:671-673 (1994); Choo & Klug, PNAS 91:11163-11167 (1994); Choo & Klug, PNAS 91: 11168-11172 (1994); Desjarlais & Berg, PNAS 90:2256-2260 (1993); Desjarlais & Berg, PNAS 89:7345-7349 (1992); Pomerantz et al., Science 267:93-96 (1995); Pomerantz et al., PNAS 92:9752-9756 (1995); and Liu et al., PNAS 94:5525-5530 (1997); Griesman & Pabo, Science 275:657-661 (1997); Desjarlais & Berg, PNAS 91:11-99-11103 (1994)).

In certain preferred embodiments, the binding specificity of a DNA-binding domain (e.g., a ZFP DNA-binding domain) is determined by identifying accessible regions in the sequence in question (e.g., in cellular chromatin). Accessible regions can be determined as described in co-owned WO 01/83732. *See, also*, U.S. Patent Application No. 20030021776A1. A DNA-binding domain is then designed and/or selected as described herein to bind to a target site within the accessible region.

### VI. EXEMPLARY ZINC FINGER PROTEINS AND EQUIVALENTS

Disclosed herein are compositions and methods for regulation of transcription, which are useful, for example, for treatment of neurodegenerative conditions and neuropathies. These include fusion proteins comprising an engineered zinc finger protein and a functional domain such as, for example, a transcriptional activation domain. Suitable functional domains are known in the art and include, without limitation, transcriptional activation domains such as, for example, VP16, VP64 and p65. Moreover, one or more of the same or different functional domains (*e.g*., transcriptional activation domains) can be present in a given fusion protein. *See* co-owned U.S. Patent Application Publication No. 2002/0160940, incorporated by reference, for disclosure of exemplary transcriptional activation and repression domains.

In certain embodiments, a zinc finger protein is engineered to bind to the target sequence GGGGGTGAC (SEQ ID NO:26), which is present in the VEGF-A gene. An exemplary three-finger zinc finger protein, VOP32E, has been so engineered. The recognition regions of the three zinc fingers of VOP32E have the following amino acid sequences:
F1: DRSNLTR (SEQ ID NO:55)
F2: TSGHLSR (SEQ ID NO:141)
F3: RSDHLSR (SEQ ID NO:68).

These amino acid sequences correspond to residues -1 through +6 with respect to the start of the helical portion of a zinc finger and are denoted the "recognition regions" because one or more of these residues participate in sequence specificity of nucleic acid binding. Accordingly, proteins comprising the same three recognition regions in a different polypeptide backbone sequence are considered equivalents to the VOP32E protein, since they will have the same DNA-binding specificity. Thus, in certain embodiments, the three recognition regions (SEQ ID NOS:55, 141 and 68) can be placed in any zinc finger backbone (see, *e.g*., U.S. Patents 6,453,242 and 6,534,261) and the resulting protein can be used to regulate VEGF transcription, *e.g.*, to promote neural regeneration. Accordingly, engineered zinc finger proteins comprising the following sequence can be used in the disclosed methods:
C-X₂₋₄-C-X₅-D-R-S-N-L-T-R-H-X₃₋₅-H-X₇-C-X₂₋₄-C-X₅-T-S-G-H-L-S-R-H-X₃₋₅-H-X₇-C-X₂₋₄-C-X₅-R-S-D-H-L-S-R-H-X₃₋₅-H (SEQ ID NO:162).

Within the recognition region, residues -1, +3 and +6 are primarily responsible for protein-nucleotide contacts. Accordingly, non-limiting examples of additional equivalents include proteins comprising three zinc fingers wherein the first finger contains a D residue at -1, a N residue at +3 and a R residue at +6 (DXXNXXR, SEQ ID NO:163); the second finger contains a T residue at -1, a H residue at +3 and a R residue at +6 (TXXHXXR, SEQ ID NO:164); and the third finger contains a R residue at -1, a H residue at +3 and a R residue at +6 (RXXHXXR, SEQ ID NO:165). Thus, for example, proteins comprising SEQ ID NO:166 are considered equivalents for use in the disclosed methods.
C-X₂₋₄-C-X₅-D-X-X-N-X-X-R-H-X₃₋₅-H-X₇-C-X₂₋₄-C-X₅-T-X-X-H-X-X-R-H-X₃₋₅-H-X₇-C-X₂₋₄-C-X₅-R-X-X-H-X-X-R-H-X₃₋₅-H (SEQ ID NO:166)

Additional equivalents comprise any ZFP that binds to a sequence comprising the target sequence GGGGGTGAC (SEQ ID NO:26).

Correspondences between amino acids at the -1, +3 and +6 contact residues of the recognition region of a zinc finger, and nucleotides in a target site, have been described. See, for example, U.S. Patent Nos. 6,007,988; 6,013,453; 6,746,838 and 6,866,997; as well as PCT Publications WO 96/06166; WO 98/53058; WO 98/53059 and WO 98/53060. Accordingly, also to be considered equivalents are three-finger zinc finger proteins in which the first finger contains D or H at -1 ; N at +3 and R, K, S or T at +6 (and if S or T, also contains D at position +2 of the adjacent C-terminal zinc finger); the second finger contains H, T, N or Q at -1; H at +3 and R, K, S or T at +6 (and if S or T, also contains D at position +2 of the adjacent C-terminal zinc finger); and the third finger contains R at-1; H at +3 and R, K, S or T at +6 (and if S or T, also contains D at position +2 of the adjacent C-terminal zinc finger).

### VII. PRODUCTION OF ZINC FINGER PROTEINS

### A. SYNTHESIS AND CLONING

ZFP polypeptides and nucleic acids encoding the same can be made using routine techniques in the field of recombinant genetics. Basic texts disclosing general methods include Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994)). In addition, nucleic acids less than about 100 bases can be custom ordered from any of a variety of commercial sources, such as The Midland Certified Reagent Company (mcrc@oligos.com), The Great American Gene Company (http://www.genco.com), ExpressGen Inc. (www.expressgen.com), Operon Technologies Inc. (Alameda, Calif.). Similarly, peptides can be custom ordered from any of a variety of sources, such as PeptidoGenic (pkim@ccnet.com), HTI Bio-products, inc. (http://www.htibio.com), BMA Biomedicals Ltd (U.K.), Bio.Synthesis, Inc.

Oligonucleotides can be chemically synthesized according to the solid phase phosphoramidite triester method first described by Beaucage & Caruthers, Tetrahedron Letts. 22:1859-1862 (1981), using an automated synthesizer, as described in Van Devanter et al., Nucleic Acids Res. 12:6159-6168 (1984). Purification of oligonucleotides is by either denaturing polyacrylamide gel electrophoresis or by reverse phase HPLC. The sequence of the cloned genes and synthetic oligonucleotides can be verified after cloning using, e.g., the chain termination method for sequencing double-stranded templates of Wallace et al., Gene 16:21-26 (1981).

Two alternative methods are typically used to create the coding sequences required to express newly designed DNA-binding peptides. One protocol is a PCR-based assembly procedure that utilizes six overlapping oligonucleotides. Three oligonucleotides correspond to "universal" sequences that encode portions of the DNA-binding domain between the recognition helices. These oligonucleotides typically remain constant for all zinc finger constructs. The other three "specific" oligonucleotides are designed to encode the recognition helices. These oligonucleotides contain substitutions primarily at positions -1, 2, 3 and 6 on the recognition helices making them specific for each of the different DNA-binding domains.

The PCR synthesis is carried out in two steps. First, a double stranded DNA template is created by combining the six oligonucleotides (three universal, three specific) in a four cycle PCR reaction with a low temperature annealing step, thereby annealing the oligonucleotides to form a DNA "scaffold." The gaps in the scaffold are filled in by high-fidelity thermostable polymerase, the combination of Taq and Pfu polymerases also suffices. In the second phase of construction, the zinc finger template is amplified by external primers designed to incorporate restriction sites at either end for cloning into a shuttle vector or directly into an expression vector.

An alternative method of cloning the newly designed DNA-binding proteins relies on annealing complementary oligonucleotides encoding the specific regions of the desired ZFP. This particular application requires that the oligonucleotides be phosphorylated prior to the final ligation step. This is usually performed before setting up the annealing reactions. In brief, the "universal" oligonucleotides encoding the constant regions of the proteins (oligos 1, 2 and 3 of above) are annealed with their complementary oligonucleotides. Additionally, the "specific" oligonucleotides encoding the finger recognition helices are annealed with their respective complementary oligonucleotides. These complementary oligos are designed to fill in the region that was previously filled in by polymerase in the above-mentioned protocol. Oligonucleotides complementary to oligos 1 and 6 are engineered to leave overhanging sequences specific for the restriction sites used in cloning into the vector of choice in the following step. The second assembly protocol differs from the initial protocol in the following aspects: the "scaffold" encoding the newly designed ZFP is composed entirely of synthetic DNA thereby eliminating the polymerase fill-in step, additionally the fragment to be cloned into the vector does not require amplification. Lastly, the design of leaving sequence-specific overhangs eliminates the need for restriction enzyme digests of the inserting fragment. Alternatively, changes to ZFP recognition helices can be created using conventional site-directed mutagenesis methods.

Both assembly methods require that the resulting fragment encoding the newly designed ZFP be ligated into a vector. Ultimately, the ZFP-encoding sequence is cloned into an expression vector. Expression vectors that are commonly utilized include, but are not limited to, a modified pMAL-c2 bacterial expression vector (New England BioLabs, Beverly, Mass.) or an eukaryotic expression vector, pcDNA (Promega, Madison, Wis.). The final constructs are verified by sequence analysis.

Any suitable method of protein purification known to those of skill in the art can be used to purify ZFPs (see, Ausubel, supra, Sambrook, supra). In addition, any suitable host can be used for expression, e.g., bacterial cells, insect cells, yeast cells, mammalian cells, and the like.

Expression of a zinc finger protein fused to a maltose binding protein (MBP-ZFP) in bacterial strain JM109 allows for straightforward purification through an amylose column (New England BioLabs, Beverly, Mass.). High expression levels of the zinc finger chimeric protein can be obtained by induction with IPTG since the MBP-ZFP fusion in the pMal-c2 expression plasmid is under the control of the tac promoter (New England BioLabs, Beverly, Mass.). Bacteria containing the MBP-ZFP fusion plasmids are inoculated into 2xYT medium containing 10 µM ZnCl₂, 0.02% glucose, plus 50 µg/ml ampicillin and shaken at 37°C. At mid-exponential growth IPTG is added to 0.3 mM and the cultures are allowed to shake. After 3 hours the bacteria are harvested by centrifugation, disrupted by sonication or by passage through a pressure cell or through the use of lysozyme, and insoluble material is removed by centrifugation. The MBP-ZFP proteins are captured on an amylose-bound resin, washed extensively with buffer containing 20 mM Tris-HCl (pH 7.5), 200 mM NaCl, 5 mM DTT and 50 µM ZnCl₂, then eluted with maltose in essentially the same buffer (purification is based on a standard protocol from New England BioLabs. Purified proteins are quantitated and stored for biochemical analysis.

The dissociation constant of a purified protein, e.g., Kd, is typically characterized via electrophoretic mobility shift assays (EMSA) (Buratowski & Chodosh, in Current Protocols in Molecular Biology pp. 12.2.1-12.2.7 (Ausubel ed., 1996)). Affinity is measured by titrating purified protein against a fixed amount of labeled double-stranded oligonucleotide target. The target typically comprises the natural binding site sequence flanked by the 3 bp found in the natural sequence and additional, constant flanking sequences. The natural binding site is typically 9 bp for a three-finger protein and 2.times.9 bp +intervening bases for a six finger ZFP. The annealed oligonucleotide targets possess a 1 base 5' overhang that allows for efficient labeling of the target with T4 phage polynucleotide kinase. For the assay the target is added at a concentration of 1 nM or lower (the actual concentration is kept at least 10-fold lower than the expected dissociation constant), purified ZFPs are added at various concentrations, and the reaction is allowed to equilibrate for at least 45 min. In addition the reaction mixture also contains 10 mM Tris (pH 7.5), 100 mM KCl, 1 mM MgCl₂, 0.1 mM ZnCl₂, 5 mM DTT, 10% glycerol, 0.02% BSA.

The equilibrated reactions are loaded onto a 10% polyacrylamide gel, which has been pre-run for 45 min in Tris/glycine buffer, then bound and unbound labeled target is resolved by electrophoresis at 150V. Alternatively, 10-20% gradient Tris-HCl gels, containing a 4% polyacrylamide stacking gel, can be used. The dried gels are visualized by autoradiography or phosphorimaging and the apparent Kd is determined by calculating the protein concentration that yields half-maximal binding.

The assays can also include a determination of the active fraction in the protein preparations. Active fraction is determined by stoichiometric gel shifts in which protein is titrated against a high concentration of target DNA. Titrations are done at 100, 50, and 25% of target (usually at micromolar levels).

### B. PHAGE DISPLAY

The technique of phage display provides a largely empirical means of generating zinc finger proteins with desired target specificity (see e.g., Rebar, US 5,789,538; Choo et al., WO 96/06166; Barbas et al., WO 95/19431 and WO 98/543111; Jamieson et al., supra). The method can be used in conjunction with, or as an alternative to rational design. The method involves the generation of diverse libraries of mutagenized zinc finger proteins, followed by the isolation of proteins with desired DNA-binding properties using affinity selection methods. To use this method, the experimenter typically proceeds as follows. First, a gene for a zinc finger protein is mutagenized to introduce diversity into regions important for binding specificity and/or affinity. In a typical application, this is accomplished via randomization of a single finger at positions -1,+2,+3, and +6, and sometimes accessory positions such as +1, +5,+8 and +10. Next, the mutagenized gene is cloned into a phage or phagemid vector as a fusion with gene III of a filamentous phage, which encodes the coat protein pIII. The zinc finger gene is inserted between segments of gene III encoding the membrane export signal peptide and the remainder of pIII, so that the zinc finger protein is expressed as an amino-terminal fusion with pIII in the mature, processed protein.

When using phagemid vectors, the mutagenized zinc finger gene may also be fused to a truncated version of gene III encoding, minimally, the C-terminal region required for assembly of pIII into the phage particle. The resultant vector library is transformed into E. coli and used to produce filamentous phage that express variant zinc finger proteins on their surface as fusions with the coat protein pIII. If a phagemid vector is used, then this step requires superinfection with helper phage. The phage library is then incubated with a target DNA site, and affinity selection methods are used to isolate phage that bind target with high affinity from bulk phage. Typically, the DNA target is immobilized on a solid support, which is then washed under conditions sufficient to remove all but the tightest binding phage. After washing, any phage remaining on the support are recovered via elution under conditions which disrupt zinc finger--DNA binding. Recovered phage are used to infect fresh *E. coli,* which is then amplified and used to produce a new batch of phage particles. Selection and amplification are then repeated as many times as is necessary to enrich the phage pool for tight binders such that these may be identified using sequencing and/or screening methods. Although the method is illustrated for pIII fusions, analogous principles can be used to screen ZFP variants as pVIII fusions.

In certain embodiments, the sequence bound by a particular zinc finger protein is determined by conducting binding reactions (see, e.g., conditions for determination of Kd, supra) between the protein and a pool of randomized double-stranded oligonucleotide sequences. The binding reaction is analyzed by an electrophoretic mobility shift assay (EMSA), in which protein-DNA complexes undergo retarded migration in a gel and can be separated from unbound nucleic acid. Oligonucleotides that have bound the finger are purified from the gel and amplified, for example, by a polymerase chain reaction. The selection (i.e. binding reaction and EMSA analysis) is then repeated as many times as desired, with the selected oligonucleotide sequences. In this way, the binding specificity of a zinc finger protein having a particular amino acid sequence is determined.

### C. REGULATORY DOMAINS

Zinc finger proteins are often expressed with an exogenous domain (or functional fragment thereof) as fusion proteins. Common domains for addition to the ZFP include, e.g., transcription factor domains (activators, repressors, co-activators, co-repressors), silencers, oncogenes (e.g., myc, jun, fos, myb, max, mad, rel, ets, bcl, myb, mos family members etc.); DNA repair enzymes and their associated factors and modifiers; DNA rearrangement enzymes and their associated factors and modifiers; chromatin associated proteins and their modifiers (e.g. kinases, acetylases and deacetylases); and DNA modifying enzymes (e.g., methyltransferases, topoisomerases, helicases, ligases, kinases, phosphatases, polymerases, endonucleases) and their associated factors and modifiers. A preferred domain for fusing with a ZFP when the ZFP is to be used for repressing expression of a target gene is a KRAB repression domain from the human KOX-1 protein (Thiesen et al., New Biologist 2, 363-374 (1990); Margolin et al., Proc. Natl. Acad. Sci. USA 91, 4509-4513 (1994); Pengue et al., Nucl. Acids Res. 22:2908-2914 (1994); Witzgall et al., Proc. Natl. Acad. Sci. USA 91, 4514-4518 (1994). Preferred domains for achieving activation include the HSV VP16 activation domain (see, e.g., Hagmann et al., J. Virol. 71, 5952-5962 (1997)) nuclear hormone receptors (see, e.g., Torchia et al., Curr. Opin. Cell. Biol. 10:373-383 (1998)); the p65 subunit of nuclear factor kappa B (Bitko & Barik, J. Virol. 72:5610-5618 (1998) and Doyle & Hunt, Neuroreport 8:2937-2942 (1997)); Liu et al., Cancer Gene Ther. 5:3-28 (1998)), or artificial chimeric functional domains such as VP64 (Seifpal et al., EMBO J. 11, 4961-4968 (1992)).

The identification of novel sequences and accessible regions (e.g., DNase I hypersensitive sites) in genes associated with diabetic neuropathy (e.g., VEGF) allows for the design of fusion molecules for the treatment of diabetic neuropathy. Thus, in certain embodiments, the compositions and methods disclosed herein involve fusions between a DNA-binding domain specifically targeted to one or more regulatory regions of a VEGF gene and a functional (e.g., repression or activation) domain (or a polynucleotide encoding such a fusion). In this way, the repression or activation domain is brought into proximity with a sequence in the gene that is bound by the DNA-binding domain. The transcriptional regulatory function of the functional domain is then able to act on the selected regulatory sequences. *See, e.g.,* US Patent Application Publication No. 2002-0064802.

In additional embodiments, targeted remodeling of chromatin, as disclosed in co-owned WO 01/83793 can be used to generate one or more sites in cellular chromatin that are accessible to the binding of a DNA binding molecule.

Fusion molecules are constructed by methods of cloning and biochemical conjugation that are well known to those of skill in the art. Fusion molecules comprise a DNA-binding domain and a functional domain (e.g., a transcriptional activation or repression domain). Fusion molecules also optionally comprise nuclear localization signals (such as, for example, that from the SV40 medium T-antigen) and epitope tags (such as, for example, FLAG and hemagglutinin). Fusion proteins (and nucleic acids encoding them) are designed such that the translational reading frame is preserved among the components of the fusion.

Fusions between a polypeptide component of a functional domain (or a functional fragment thereof) on the one hand, and a non-protein DNA-binding domain (e.g., antibiotic, intercalator, minor groove binder, nucleic acid) on the other, are constructed by methods of biochemical conjugation known to those of skill in the art. See, for example, the Pierce Chemical Company (Rockford, IL) Catalogue. Methods and compositions for making fusions between a minor groove binder and a polypeptide have been described. Mapp et al. (2000) Proc. Natl. Acad. Sci. USA 97:3930-3935.

In certain embodiments, the target site bound by the zinc finger protein is present in an accessible region of cellular chromatin. Accessible regions can be determined as described, for example, in co-owned International Publication WO 01/83732. If the target site is not present in an accessible region of cellular chromatin, one or more accessible regions can be generated as described in co-owned WO 01/83793. In additional embodiments, the DNA-binding domain of a fusion molecule is capable of binding to cellular chromatin regardless of whether its target site is in an accessible region or not. For example, such DNA-binding domains are capable of binding to linker DNA and/or nucleosomal DNA. Examples of this type of "pioneer" DNA binding domain are found in certain steroid receptor and in hepatocyte nuclear factor 3 (HNF3). Cordingley et al. (1987) Cell 48:261-270; Pina et al. (1990) Cell 60:719-731; and Cirillo et al. (1998) EMBO J. 17:244-254.

For such applications, the fusion molecule is typically formulated with a pharmaceutically acceptable carrier, as is known to those of skill in the art. See, for example, Remington's Pharmaceutical Sciences, 17th ed., 1985; and co-owned WO 00/42219.

The functional component/domain of a fusion molecule can be selected from any of a variety of different components capable of influencing transcription of a gene once the fusion molecule binds to a target sequence via its DNA binding domain. Hence, the functional component can include, but is not limited to, various transcription factor domains, such as activators, repressors, co-activators, co-repressors, and silencers.

Exemplary functional domains for fusing with a DNA-binding domain such as, for example, a ZFP, to be used for repressing expression of a gene are the KOX repression domain and the KRAB repression domain from the human KOX-1 protein (see, e.g., Thiesen et al., New Biologist 2, 363-374 (1990); Margolin et al., Proc. Natl. Acad. Sci. USA 91, 4509-4513 (1994); Pengue et al., Nucl. Acids Res. 22:2908-2914 (1994); Witzgall et al., Proc. Natl. Acad. Sci. USA 91, 4514-4518 (1994). Another suitable repression domain is methyl binding domain protein 2B (MBD-2B) (see, also Hendrich et al. (1999) Mamm Genome 10:906-912 for description of MBD proteins). Another useful repression domain is that associated with the v-ErbA protein. See, for example, Damm, et al. (1989) Nature 339:593-597; Evans (1989) Int. J. Cancer Suppl. 4:26-28; Pain et al. (1990) New Biol. 2:284-294; Sap et al. (1989) Nature 340:242-244; Zenke et al. (1988) Cell 52:107-119; and Zenke et al. (1990) Cell 61:1035-1049.

Suitable domains for achieving activation include the HSV VP16 activation domain (see, e.g., Hagmann et al., J. Virol. 71, 5952-5962 (1997)) nuclear hormone receptors (see, e.g., Torchia et al., Curr. Opin. Cell. Biol. 10:373-383 (1998)); the p65 subunit of nuclear factor kappa B (Bitko & Barik, J. Virol. 72:5610-5618 (1998) and Doyle & Hunt, Neuroreport 8:2937-2942 (1997)); Liu et al., Cancer Gene Ther. 5:3-28 (1998)), or artificial chimeric functional domains such as VP64 (Seifpal et al., EMBO J. 11, 4961-4968 (1992)). Additional exemplary activation domains include, but are not limited to, VP16, VP64, p300, CBP, PCAF, SRC1 PvALF, AtHD2A and ERF-2. See, for example, Robyr et al. (2000) Mol. Endocrinol. 14:329-347; Collingwood et al. (1999) J. Mol. Endocrinol. 23:255-275; Leo et al. (2000) Gene 245:1-11; Manteuffel-Cymborowska (1999) Acta Biochim. Pol. 46:77-89; McKenna et al. (1999) J. Steroid Biochem. Mol. Biol. 69:3-12; Malik et al. (2000) Trends Biochem. Sci. 25:277-283; and Lemon et al. (1999) Curr. Opin. Genet. Dev. 9:499-504. Additional exemplary activation domains include, but are not limited to, OsGAI, HALF-1, C1, API, ARF-5,-6,-7, and -8, CPRF1, CPRF4, MYC-RP/GP, and TRAB1. See, for example, Ogawa et al. (2000) Gene 245:21-29; Okanami et al. (1996) Genes Cells 1:87-99; Goff et al. (1991) Genes Dev. 5:298-309; Cho et al. (1999) Plant Mol. Biol. 40:419-429; Ulmason et al. (1999) Proc. Natl. Acad. Sci. USA 96:5844-5849; Sprenger-Haussels et al. (2000) Plant J. 22:1-8; Gong et al. (1999) Plant Mol. Biol. 41:33-44; and Hobo et al. (1999) Proc. Natl. Acad. Sci. USA 96:15,348-15,353.

Additional exemplary repression domains include, but are not limited to, KRAB, KOX, SID, MBD2, MBD3, members of the DNMT family (e.g., DNMT1, DNMT3A, DNMT3B), Rb, and MeCP2. See, for example, Bird et al. (1999) Cell 99:451-454; Tyler et al. (1999) Cell 99:443-446; Knoepfler et al. (1999) Cell 99:447-450; and Robertson et al. (2000) Nature Genet. 25:338-342. Additional exemplary repression domains include, but are not limited to, ROM2 and AtHD2A. See, for example, Chem et al. (1996) Plant Cell 8:305-321; and Wu et al. (2000) Plant J. 22:19-27.

Additional exemplary functional domains are disclosed, for example, in co-owned US Patent No. 6,534,261 and US Patent Application Publication No. 2002/0160940.

### D. EXPRESSION VECTORS

The nucleic acid encoding the ZFP of choice is typically cloned into intermediate vectors for transformation into prokaryotic or eukaryotic cells for replication and/or expression, e.g., for determination of K_{d}. Intermediate vectors are typically prokaryote vectors, e.g., plasmids, or shuttle vectors, or insect vectors, for storage or manipulation of the nucleic acid encoding ZFP or production of protein. The nucleic acid encoding a ZFP is also typically cloned into an expression vector, for administration to a plant cell, animal cell, preferably a mammalian cell or a human cell, fungal cell, bacterial cell, or protozoal cell.

To obtain expression of a cloned gene or nucleic acid, a ZFP is typically subcloned into an expression vector that contains a promoter to direct transcription. Suitable bacterial and eukaryotic promoters are well known in the art and described, e.g., in Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994). Bacterial expression systems for expressing the ZFP are available in, e.g., E. coli, Bacillus sp., and Salmonella (Palva et al., Gene 22:229-235 (1983)). Kits for such expression systems are commercially available. Eukaryotic expression systems for mammalian cells, yeast, and insect cells are well known in the art and are also commercially available.

The promoter used to direct expression of a ZFP nucleic acid depends on the particular application. For example, a strong constitutive promoter is typically used for expression and purification of ZFP. In contrast, when a ZFP is administered in vivo for gene regulation, either a constitutive or an inducible promoter is used, depending on the particular use of the ZFP. In addition, a preferred promoter for administration of a ZFP can be a weak promoter, such as HSV TK or a promoter having similar activity. The promoter typically can also include elements that are responsive to transactivation, e.g., hypoxia response elements, Gal4 response elements, lac repressor response element, and small molecule control systems such as tet-regulated systems and the RU-486 system (see, e.g., Gossen & Bujard, PNAS 89:5547 (1992); Oligino et al., Gene Ther. 5:491-496 (1998); Wang et al., Gene Ther. 4:432-441 (1997); Neering et al., Blood 88:1147-1155 (1996); and Rendahl et al., Nat. Biotechnol. 16:757-761 (1998)).

In addition to the promoter, the expression vector typically contains a transcription unit or expression cassette that contains all the additional elements required for the expression of the nucleic acid in host cells, either prokaryotic or eukaryotic. A typical expression cassette thus contains a promoter operably linked, e.g., to the nucleic acid sequence encoding the ZFP, and signals required, e.g., for efficient polyadenylation of the transcript, transcriptional termination, ribosome binding sites, or translation termination. Additional elements of the cassette may include, e.g., enhancers, and exogenous spliced intronic signals.

The particular expression vector used to transport the genetic information into the cell is selected with regard to the intended use of the ZFP. Standard bacterial expression vectors include plasmids such as pBR322 based plasmids, pSKF, pET23D, and commercially available fusion expression systems such as GST and LacZ. A preferred fusion protein is the maltose binding protein, "MBP." Such fusion proteins are used for purification of the ZFP. Epitope tags can also be added to recombinant proteins to provide convenient methods of isolation, for monitoring expression, and for monitoring cellular and subcellular localization, e.g., c-myc or FLAG.

Expression vectors containing regulatory elements from eukaryotic viruses are often used in eukaryotic expression vectors, e.g., SV40 vectors, papilloma virus vectors, and vectors derived from Epstein-Barr virus. Other exemplary eukaryotic vectors include pMSG, pAV009/A+, pMTO10/A+, pMAMneo-5, baculovirus pDSVE, and any other vector allowing expression of proteins under the direction of the SV40 early promoter, SV40 late promoter, metallothionein promoter, murine mammary tumor virus promoter, Rous sarcoma virus promoter, polyhedrin promoter, or other promoters shown effective for expression in eukaryotic cells.

Some expression systems have markers for selection of stably transfected cell lines such as thymidine kinase, hygromycin B phosphotransferase, and dihydrofolate reductase. High yield expression systems are also suitable, such as using a baculovirus vector in insect cells, with a ZFP encoding sequence under the direction of the polyhedrin promoter or other strong baculovirus promoters.

The elements that are typically included in expression vectors also include a replicon that functions in *E. coli,* a gene encoding antibiotic resistance to permit selection of bacteria that harbor recombinant plasmids, and unique restriction sites in nonessential regions of the plasmid to allow insertion of recombinant sequences.

Standard transfection methods are used to produce bacterial, mammalian, yeast or insect cell lines that express large quantities of protein, which are then purified using standard techniques (see, e.g., Colley et al., J. Biol. Chem. 264:17619-17622 (1989); Guide to Protein Purification, in Methods in Enzymology, vol. 182 (Deutscher, ed., 1990)). Transformation of eukaryotic and prokaryotic cells are performed according to standard techniques (see, e.g., Morrison, J. Bact. 132:349-351 (1977); Clark-Curtiss & Curtiss, Methods in Enzymology 101:347-362 (Wu et al., eds, 1983).

Any of the well known procedures for introducing foreign nucleotide sequences into host cells may be used. These include the use of calcium phosphate transfection, polybrene, protoplast fusion, electroporation, liposomes, microinjection, naked DNA, plasmid vectors, viral vectors, both episomal and integrative, and any of the other well known methods for introducing cloned genomic DNA, cDNA, synthetic DNA or other foreign genetic material into a host cell (see, e.g., Sambrook et al., supra). It is only necessary that the particular genetic engineering procedure used be capable of successfully introducing at least one gene into the host cell capable of expressing the protein of choice.

### VIII. ASSAYS

Once a ZFP has been designed and prepared according to the procedures just set forth, an initial assessment of the activity of the designed ZFP is undertaken. ZFP proteins showing the ability to modulate the expression of a gene of interest can then be further assayed for more specific activities depending upon the particular application for which the ZFPs have been designed. Thus, for example, the ZFPs provided herein can be initially assayed for their ability to modulate VEGF expression. More specific assays of the ability of the ZFP to modulate expression of the target gene to treat diabetic neuropathy are then typically undertaken. A description of these more specific assays are set forth infra in section IX.

The activity of a particular ZFP can be assessed using a variety of in vitro and in vivo assays, by measuring, e.g., protein or mRNA levels, product levels, enzyme activity, tumor growth; transcriptional activation or repression of a reporter gene; second messenger levels (e.g., cGMP, cAMP, IP3, DAG, Ca2+); cytokine and hormone production levels; and neovascularization, using, e.g., immunoassays (e.g., ELISA and immunohistochemical assays with antibodies), hybridization assays (e.g., RNase protection, RNA blots ("Northerns"), in situ hybridization, oligonucleotide array studies), colorimetric assays, amplification assays, enzyme activity assays, tumor growth assays, phenotypic assays, and the like.

ZFPs are typically first tested for activity in vitro using cultured cells, e.g., 293 cells, CHO cells, VERO cells, BHK cells, HeLa cells, COS cells, and the like. Preferably, human cells are used. The ZFP is often first tested using a transient expression system with a reporter gene, and then regulation of the target endogenous gene is tested in cells and in animals, both in vivo and ex vivo. The ZFP can be recombinantly expressed in a cell, recombinantly expressed in cells transplanted into an animal, or recombinantly expressed in a transgenic animal, as well as administered as a protein to an animal or cell using delivery vehicles described below. The cells can be immobilized, be in solution, be injected into an animal, or be naturally occurring in a transgenic or non-transgenic animal.

Modulation of gene expression is tested using one of the in vitro or in vivo assays described herein. Samples or assays are treated with a ZFP and compared to untreated control samples, to examine the extent of modulation. As described above, for regulation of endogenous gene expression, the ZFP typically has a Kd of 200 nM or less, more preferably 100 nM or less, more preferably 50 nM, most preferably 25 nM or less.

The effects of the ZFPs can be measured by examining any of the parameters described above. Any suitable gene expression, phenotypic, or physiological change can be used to assess the influence of a ZFP. When the functional consequences are determined using intact cells or animals, one can also measure a variety of effects such as neurotrophism, transcriptional changes to both known and uncharacterized genetic markers (e.g., Northern blots or oligonucleotide array studies), changes in cell metabolism such as cell growth or pH changes, and changes in intracellular second messengers such as cAMP or cGMP.

Preferred assays for ZFP regulation of endogenous gene expression can be performed in vitro. In one preferred in vitro assay format, ZFP regulation of endogenous gene expression in cultured cells is measured by examining protein production using an ELISA assay. The test sample is compared to control cells treated with a vector lacking ZFP-encoding sequences or a vector encoding an unrelated ZFP that is targeted to another gene.

In another embodiment, ZFP regulation of endogenous gene expression is determined in vitro by measuring the level of target gene mRNA expression. The level of gene expression is measured using amplification, e.g., using PCR, LCR, or hybridization assays, e.g., Northern hybridization, dot blotting and RNase protection. The use of quantitative RT-PCR techniques (i.e., the so-called TaqMan assays) can also be utilized to quantitate the level of transcript. The level of protein or mRNA is detected using directly or indirectly labeled detection agents, e.g., fluorescently or radioactively labeled nucleic acids, radioactively or enzymatically labeled antibodies, and the like, as described herein. Such methods are also described in U.S. Pat Nos. 5,210,015 to Gelfand, U.S. Pat. No. 5,538,848 to Livak, et al., and U.S. Pat. No. 5,863,736 to Haaland, as well as Heid, C. A., et al., Genome Research, 6:986-994 (1996); Gibson, U. E. M, et al., Genome Research 6:995-1001 (1996); Holland, P. M., et al., Proc. Natl. Acad. Sci. USA 88:7276-7280, (1991); and Livak, K. J., et al., PCR Methods and Applications 357-362 (1995), each of which is incorporated by reference in its entirety.

Alternatively, a reporter gene system can be devised using a gene promoter from the selected target gene (*e.g*., VEGF) operably linked to a reporter gene such as luciferase, green fluorescent protein, CAT, or □gal. The reporter construct is typically co-transfected into a cultured cell. After treatment with the ZFP of choice, the amount of reporter gene transcription, translation, or activity is measured according to standard techniques known to those of skill in the art.

Another example of a preferred assay format useful for monitoring ZFP regulation of endogenous gene expression is performed in vivo. This assay is particularly useful for examining genes involved in nerve function. In this assay, the ZFP of choice is administered (*e.g*., intramuscular injection) into an animal exhibiting diabetic neuropathy. After a suitable length of time, preferably 4-8 weeks, motor and sensory nerve conduction velocities are compared to control animals that are also diabetic. Nerve velocities that exhibit significant differences as between control and diabetic (using, e.g., Student's T test) are said to have been affected by the ZFP. Alternatively, immunoassays using nerve cell specific antibodies used to stain for growth of nerve tissue can be used.

### IX. PHARMACEUTICAL COMPOSITIONS

The ZFPs provided herein, and more typically the nucleic acids encoding them, can optionally be formulated with a pharmaceutically acceptable carrier as a pharmaceutical composition.

### A. NUCLEIC ACID BASED COMPOSITIONS

Conventional viral and non-viral based gene transfer methods can be used to introduce nucleic acids encoding the present ZFPs in mammalian cells or target tissues. Such methods can be used to administer nucleic acids encoding ZFPs to cells in vitro. In some instances, the nucleic acids encoding ZFPs are administered for in vivo or ex vivo gene therapy uses. Non-viral vector delivery systems include DNA plasmids, naked nucleic acid, and nucleic acid complexed with a delivery vehicle such as a poloxamer or liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11: 167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Bohm (eds) (1995); and Yu et al., Gene Therapy 1:13-26 (1994).

Methods of non-viral delivery of nucleic acids encoding the ZFPs provided herein include lipofection, electorporation, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam^{™}. and Lipofectin^{™}.). Cationic and neutral lipids that are suitable for efficient receptor-recognition lipofection of polynucleotides include those of Felgner, WO 91/17424, WO 91/16024. Delivery can be to cells (ex vivo administration) or target tissues (in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids encoding engineered ZFP take advantage of highly evolved processes for targeting a virus to specific cells in the body and trafficking the viral payload to the nucleus. Viral vectors can be administered directly to patients (in vivo) or they can be used to treat cells in vitro and the modified cells are administered to patients (ex vivo). Conventional viral based systems for the delivery of ZFPs can include retroviral, lentivirus, adenoviral, adeno-associated and herpes simplex virus vectors for gene transfer. Viral vectors are currently the most efficient and versatile method of gene transfer in target cells and tissues. Integration in the host genome is possible with the retrovirus, lentivirus, and adeno-associated virus gene transfer methods, often resulting in long-term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vector that are able to transduce or infect non-dividing cells and typically produce high viral titers. Selection of a retroviral gene transfer system can therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In applications where transient expression of the ZFP is preferred, adenoviral based systems are typically used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors are also used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

In particular, at least six viral vector approaches are currently available for gene transfer in clinical trials, with retroviral vectors by far the most frequently used system. All of these viral vectors utilize approaches that involve complementation of defective vectors by genes inserted into helper cell lines to generate the transducing agent.

pLASN and MFG-S are examples are retroviral vectors that have been used in clinical trials (Dunbar et al., Blood 85:3048-305 (1995); Kohn et al., Nat. Med. 1:1017-102 (1995); Malech et al., PNAS 94:22 12133-12138 (1997)). PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol Immunother. 44(1):10-20 (1997); Dranoff et al., Hum. Gene Ther. 1:111-2 (1997).

Recombinant adeno-associated virus vectors (rAAV) is another alternative gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:9117 1702-3 (1998), Kearns et al., Gene Ther. 9:748-55 (1996)).

Replication-deficient recombinant adenoviral vectors (Ad) are predominantly used for colon cancer gene therapy, because they can be produced at high titer and they readily infect a number of different cell types. Most adenovirus vectors are engineered such that a transgene replaces the Ad E1a, E1b, and E3 genes; subsequently the replication defector vector is propagated in human 293 cells that supply deleted gene function in trans. Ad vectors can transduce multiply types of tissues in vivo, including nondividing, differentiated cells such as those found in the liver, kidney and muscle system tissues. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topfet al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and .psi.2 cells or PA317 cells, which package retrovirus. Viral vectors used in gene therapy are usually generated by producer cell line that packages a nucleic acid vector into a viral particle. The vectors typically contain the minimal viral sequences required for packaging and subsequent integration into a host, other viral sequences being replaced by an expression cassette for the protein to be expressed. The missing viral functions are supplied in trans by the packaging cell line. For example, AAV vectors used in gene therapy typically only possess ITR sequences from the AAV genome that are required for packaging and integration into the host genome. Viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

As stated above, various viral delivery vehicles, as are known in the art, can be used to introduce a nucleic acid (e.g., a nucleic acid encoding a zinc finger protein) into a cell. The choice of delivery vehicle depends upon a number of factors, including but not limited to the size of the nucleic acid to be delivered and the desired target cell.

In certain embodiments, adenoviruses are used as delivery vehicles. Exemplary adenovirus vehicles include Adenovirus Types 2, 5, 12 and 35. For example, vehicles useful for introduction of transgenes into hematopoietic stem cells, *e.g*., CD34⁺ cells, include adenovirus Type 35. Additional adenoviral vehicles include the so-called "gutless" adenoviruses. *See,* for example, Kochanek et al. (1996) Proc. Natl. Acad. Sci. USA 93:5,731-5,736.

Adeno-associated virus vehicles include AAV serotypes 1, 2, 5, 6, 7, 8 and 9; as well as chimeric AAV serotypes, *e.g*., AAV 2/1 and AAV 2/5 Both single- and double-stranded AAV vectors can be used.

Lentivirus delivery vehicles have been described, for example, in U.S. Patents 6,312,682 and 6,669,936 and in U.S. Patent Application Publication No. 2002/0173030 and can be used, *e.g.*, to introduce transgenes into immune cells (*e.g*., T-cells). Lentiviruses are capable of integrating a DNA copy of their RNA genome into the genome of a host cell. *See,* for example, Ory et al. (1996) Proc. Natl. Acad. Sci. USA 93:11382-11388; Miyoshi et al. (1998) J. Virology 72:8150-8157; Dull et al. (1998) J. Virol. 72:8463-8471; Zuffery et al. (1998) J. Virol. 72:9873-9880; Follenzi et al. (2000) Nature Genetics 25:217-222 and Delenda (2004) J. Gene Medicine 6:S125-S138. In certain lentiviral vehicles, this integration function has been disabled to generate non-integrating lentivirus vehicles. *See,* for example, Poon et al. (2003) J. Virology 77:3962-3972 and Vargas et al. (2004) Human Gene Therapy 15:361-372. The use of both integrating and non-integrating lentivirus vectors for transduction of hematopoietic stem cells has been described by Haas et al. (2000) Mol. Therapy 2:71-80. Transduction of CD4⁺ T-cells with integrating lentivirus vectors has been described by Humeau et al. (2004) Mol. Therapy 9:902-913.

Herpes simplex virus vehicles, which are capable of long-term expression in neurons and ganglia, have been described. *See,* for example, Krisky et al. (1998) Gene Therapy 5(11):1517-1530; Krisky et al. (1998) Gene Therapy 5(12):1593-1603; Burton et al. (2001) Stem Cells 19:358-377; Lilley et al. (2001) J. Virology 75(9):4343-4356.

For delivery to neural tissue, herpes simplex virus (HSV) vectors can be particularly suitable. See, for example, Coffin et al. (1996) In: Genetic Manipulation of the Nervous System (DS Latchman, Ed.) pp 99-114: Academic Press, London; Fink et al. (1996) Ann. Rev. Neurosci. 19:265-287. In particular, replication-defective HSV vectors have been described. *See, e.g.,* U.S Patents 5,849, 571; 5,849,572; 6,248,320; 6,261,552; 6,344,445; 6,613,892 6,719,982; and 6,821,753. *See* also U.S. Patent Application Publications 2002/0192802; 2003/0040500; 2003/0082142; 2003/0091537; 2003/0113348; 2003/0219409; and 2004/0063094. *See* also Krisky et al. (1998) Gene Therapy 5:1517-1530; Palmer et al. (2000) J. Virol. 74:5604-5618; Lilley et al. (2001) J. Virol. 75:4343-4356; Burton et al. (2002) Curr. Opin. Biotechnol. 13:424-428; and Goins et al. (2002) Methods Mol. Med. 69:481-507.

Methods for improving the efficiency of retroviral transduction of hematopoietic stem cells are disclosed, for example, in U.S. Patent No. 5,928,638.

The tropism of retroviral and lentiviral delivery vehicles can be altered by the process of pseudotyping, thereby enabling viral delivery of a nucleic acid to a particular cell type. *See*, for example, U.S. Patent No. 5,817,491.

In many gene therapy applications, it is desirable that the gene therapy vector be delivered with a high degree of specificity to a particular tissue type. A viral vector is typically modified to have specificity for a given cell type by expressing a ligand as a fusion protein with a viral coat protein on the viruses outer surface. The ligand is chosen to have affinity for a receptor known to be present on the cell type of interest. For example, Han et al., PNAS 92:9747-9751 (1995), reported that Moloney murine leukemia virus can be modified to express human heregulin fused to gp70, and the recombinant virus infects certain human breast cancer cells expressing human epidermal growth factor receptor. This principle can be extended to other pairs of virus expressing a ligand fusion protein and target cell expressing a receptor. For example, filamentous phage can be engineered to display antibody fragments (e.g., FAb or Fv) having specific binding affinity for virtually any chosen cellular receptor. Although the above description applies primarily to viral vectors, the same principles can be applied to nonviral vectors. Such vectors can be engineered to contain specific uptake sequences thought to favor uptake by specific target cells.

Gene therapy vectors can be delivered *in vivo* by administration to an individual patient, typically by systemic administration (*e*.*g*., intravenous, intraperitoneal, intramuscular, subdermal, or intracranial infusion) or topical application, as described below. Alternatively, vectors can be delivered to cells ex vivo, such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector.

Ex vivo cell transfection for diagnostics, research, or for gene therapy (e.g., via re-infusion of the transfected cells into the host organism) is well known to those of skill in the art. In some instances, cells are isolated from the subject organism, transfected with a ZFP nucleic acid (gene or cDNA), and re-infused back into the subject organism (e.g., patient). Various cell types suitable for ex vivo transfection are well known to those of skill in the art (see, e.g., Freshney et al., Culture of Animal Cells, A Manual of Basic Technique (3rd ed. 1994)) and the references cited therein for a discussion of how to isolate and culture cells from patients).

In one embodiment, stem cells are used in ex vivo procedures for cell transfection and gene therapy. The advantage to using stem cells is that they can be differentiated into other cell types in vitro, or can be introduced into a mammal (such as the donor of the cells) where they will engraft in the bone marrow. Methods for differentiating CD34+cells in vitro into clinically important immune cell types using cytokines such a GM-CSF, IFN-Y and TNF-□are known (see Inaba et al., J. Exp. Med. 176:1693-1702 (1992)).

Stem cells are isolated for transduction and differentiation using known methods. For example, stem cells are isolated from bone marrow cells by panning the bone marrow cells with antibodies which bind unwanted cells, such as CD4+ and CD8+ (T cells), CD45+ (panB cells), GR-1 (granulocytes), and lad (differentiated antigen presenting cells) (see Inaba et al., J. Exp. Med. 176:1693-1702 (1992)).

Vectors (e.g., retroviruses, adenoviruses, herpesviruses, liposomes, etc.) containing therapeutic ZFP nucleic acids can be also administered directly to the organism for transduction of cells in vivo. Alternatively, naked DNA can be administered. Administration is by any of the routes normally used for introducing a molecule into ultimate contact with blood or tissue cells. Suitable methods of administering such nucleic acids are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions, as described below (see, e.g., Remington's Pharmaceutical Sciences, 17th ed., 1989).

### B. PROTEIN COMPOSITIONS

An important factor in the administration of polypeptide compounds, such as the present ZFPs, is ensuring that the polypeptide has the ability to traverse the plasma membrane of a cell, or the membrane of an intra-cellular compartment such as the nucleus. Cellular membranes are composed of lipid-protein bilayers that are freely permeable to small, nonionic lipophilic compounds and are inherently impermeable to polar compounds, macromolecules, and therapeutic or diagnostic agents. However, proteins and other compounds such as liposomes have been described, which have the ability to translocate polypeptides such as ZFPs across a cell membrane.

For example, "membrane translocation polypeptides" have amphiphilic or hydrophobic amino acid subsequences that have the ability to act as membrane-translocating carriers. In one embodiment, homeodomain proteins have the ability to translocate across cell membranes. The shortest internalizable peptide of a homeodomain protein, Antennapedia, was found to be the third helix of the protein, from amino acid position 43 to 58 (see, e.g., Prochiantz, Current Opinion in Neurobiology 6:629-634 (1996)). Another subsequence, the h (hydrophobic) domain of signal peptides, was found to have similar cell membrane translocation characteristics (see, e.g., Lin et al., J. Biol. Chem. 270:14255-14258 (1995)).

Examples of peptide sequences which can be linked to a ZFP, for facilitating uptake of ZFP into cells, include, but are not limited to: an 11 amino acid peptide of the tat protein of HIV; a 20 residue peptide sequence which corresponds to amino acids 84-103 of the p16 protein (see Fahraeus et al., Current Biology 6:84 (1996)); the third helix of the 60-amino acid long homeodomain of Antennapedia (Derossi et al., J. Biol. Chem. 269:10444 (1994)); the h region of a signal peptide such as the Kaposi fibroblast growth factor (K-FGF) h region (Lin et al., supra); or the VP22 translocation domain from HSV (Elliot & O'Hare, Cell 88:223-233 (1997)). Other suitable chemical moieties that provide enhanced cellular uptake may also be chemically linked to ZFPs. Membrane translocation domains (*i*.*e*., internalization domains) can also be selected from libraries of randomized peptide sequences. *See*, for example, Yeh et al. (2003) Molecular Therapy 7(5):S461, Abstract #1191.

Toxin molecules also have the ability to transport polypeptides across cell membranes. Often, such molecules are composed of at least two parts (called "binary toxins"): a translocation or binding domain or polypeptide and a separate toxin domain or polypeptide. Typically, the translocation domain or polypeptide binds to a cellular receptor, and then the toxin is transported into the cell. Several bacterial toxins, including *Clostridium perfringens* iota toxin, diphtheria toxin (DT), *Pseudomonas* exotoxin A (PE), pertussis toxin (PT), *Bacillus anthracis* toxin, and pertussis adenylate cyclase (CYA), have been used in attempts to deliver peptides to the cell cytosol as internal or amino-terminal fusions (Arora et al., J. Biol. Chem., 268:3334-3341 (1993); Perelle et al., Infect. Immun., 61:5147-5156 (1993); Stemnark et al., J. Cell Biol. 113:1025-1032 (1991); Donnelly et al., PNAS 90:3530-3534 (1993); Carbonetti et al., Abstr. Annu. Meet. Am. Soc. Microbiol. 95:295 (1995); Sebo et al., Infect. Immun. 63:3851-3857 (1995); Klimpel et al., PNAS U.S.A. 89:10277-10281 (1992); and Novak et al., J. Biol. Chem. 267:17186-17193 1992)).

Such subsequences can be used to translocate ZFPs across a cell membrane. ZFPs can be conveniently fused to or derivatized with such sequences. Typically, the translocation sequence is provided as part of a fusion protein. Optionally, a linker can be used to link the ZFP and the translocation sequence. Any suitable linker can be used, e.g., a peptide linker.

The ZFP can also be introduced into an animal cell, preferably a mammalian cell, via a liposomes and liposome derivatives such as immunoliposomes. The term "liposome" refers to vesicles comprised of one or more concentrically ordered lipid bilayers, which encapsulate an aqueous phase. The aqueous phase typically contains the compound to be delivered to the cell, i.e., a ZFP. The liposome fuses with the plasma membrane, thereby releasing the drug into the cytosol. Alternatively, the liposome is phagocytosed or taken up by the cell in a transport vesicle. Once in the endosome or phagosome, the liposome either degrades or fuses with the membrane of the transport vesicle and releases its contents.

In current methods of drug delivery via liposomes, the liposome ultimately becomes permeable and releases the encapsulated compound (in this case, a ZFP) at the target tissue or cell. For systemic or tissue specific delivery, this can be accomplished, for example, in a passive manner wherein the liposome bilayer degrades over time through the action of various agents in the body. Alternatively, active drug release involves using an agent to induce a permeability change in the liposome vesicle. Liposome membranes can be constructed so that they become destabilized when the environment becomes acidic near the liposome membrane (see, e.g., PNAS 84:7851 (1987); Biochemistry 28:908 (1989)). When liposomes are endocytosed by a target cell, for example, they become destabilized and release their contents. This destabilization is termed fusogenesis.
Dioleoylphosphatidylethanolamine (DOPE) is the basis of many "fusogenic" systems.

Such liposomes typically comprise a ZFP and a lipid component, e.g., a neutral and/or cationic lipid, optionally including a receptor-recognition molecule such as an antibody that binds to a predetermined cell surface receptor or ligand (e.g., an antigen). A variety of methods are available for preparing liposomes as described in, e.g., Szoka et al., Ann. Rev. Biophys. Bioeng. 9:467 (1980), U.S. Pat. Nos. 4,186,183, 4,217,344, 4,235,871, 4,261,975, 4,485,054, 4,501,728, 4,774,085, 4,837,028, 4,235,871, 4,261,975, 4,485,054, 4,501,728, 4,774,085, 4,837,028,4,946,787, PCT Publication No. WO 91.backslash.17424, Deamer & Bangham, Biochim. Biophys. Acta 443:629-634 (1976); Fraley, et al., PNAS 76:3348-3352 (1979); Hope et al., Biochim. Biophys. Acta 812:55-65 (1985); Mayer et al., Biochim. Biophys. Acta 858:161-168 (1986); Williams et al., PNAS 85:242-246 (1988); Liposomes (Ostro (ed.), 1983, Chapter 1); Hope et al., Chem. Phys. Lip. 40:89 (1986); Gregoriadis, Liposome Technology (1984) and Lasic, Liposomes: from Physics to Applications (1993)). Suitable methods include, for example, sonication, extrusion, high pressure/homogenization, microfluidization, detergent dialysis, calcium-induced fusion of small liposome vesicles and ether-fusion methods, all of which are well known in the art.

In some instances, liposomes are targeted using targeting moieties that are specific to a particular cell type, tissue, and the like. Targeting of liposomes using a variety of targeting moieties (e.g., ligands, receptors, and monoclonal antibodies) has been previously described (see, e.g., U.S. Pat. Nos. 4,957,773 and 4,603,044).

Standard methods for coupling targeting agents to liposomes can be used. These methods generally involve incorporation into liposomes lipid components, e.g., phosphatidylethanolamine, which can be activated for attachment of targeting agents, or derivatized lipophilic compounds, such as lipid derivatized bleomycin. Antibody targeted liposomes can be constructed using, for instance, liposomes which incorporate protein A (see Renneisen et al., J. Biol. Chem., 265:16337-16342 (1990) and Leonetti et al., PNAS 87:2448-2451 (1990).

### C. DOSAGE

For therapeutic applications of ZFPs, the dose administered to a patient should be sufficient to affect a beneficial therapeutic response in the patient over time. The dose will be determined by the efficacy and Kd of the particular ZFP employed, the nuclear volume of the target cell, and the condition of the patient, as well as the body weight or surface area of the patient to be treated. The size of the dose also will be determined by the existence, nature, and extent of any adverse side effects that accompany the administration of a particular compound or vector in a particular patient.

In determining the effective amount of the ZFP to be administered in the treatment or prophylaxis of diabetic neuropathy, the physician evaluates circulating plasma levels of the ZFP or nucleic acid encoding the ZFP, potential ZFP toxicities, progression of the disease, and the production of anti-ZFP antibodies. Administration can be accomplished via single or divided doses.

### D. COMPOSITIONS AND MODES OF ADMINISTRATION

### 1. GENERAL

ZFPs and the nucleic acids encoding the ZFPs can be administered directly to a patient for modulation of gene expression and for therapeutic or prophylactic applications. In general, and in view of the discussion herein, phrases referring to introducing a ZFP into an animal or patient can mean that a ZFP or ZFP fusion protein is introduced and/or that a nucleic acid encoding a ZFP of ZFP fusion protein is introduced in a form that can be expressed in the animal. For example, as described in greater detail in the following section, the ZFPs and/or nucleic acids can be used in the treatment of one or more neuropathies.

Administration of therapeutically effective amounts is by any of the routes normally used for introducing ZFP into ultimate contact with the tissue to be treated. The ZFPs or ZFP-encoding nucleic acids are administered in any suitable manner, preferably with pharmaceutically acceptable carriers (*e*.*g*., poloxamer and/or buffer). Suitable methods of administering such modulators are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there are a wide variety of suitable formulations of pharmaceutical compositions. *See*, *e*.*g*., Remington's Pharmaceutical Sciences, 17th ed. (1985).

The ZFPs, alone or in combination with other suitable components, can be made into aerosol formulations (i.e., they can be "nebulized") to be administered via inhalation. Aerosol formulations can be placed into pressurized acceptable propellants, such as dichlorodifluoromethane, propane, nitrogen, and the like.

Formulations suitable for parenteral administration, such as, for example, by intravenous, intramuscular, intradermal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of the disclosed methods, compositions can be administered, for example, by intravenous infusion, orally, topically, intraperitoneally, intravesically or intrathecally. The formulations of compounds can be presented in unit-dose or multi-dose sealed containers, such as ampoules and vials. Injection solutions and suspensions can be prepared from sterile powders, granules, and tablets of the kind previously described.

### 2. EXEMPLARY DELIVERY OPTIONS

A variety of delivery options are available for the delivery of the pharmaceutical compositions provided herein so as to treat diabetic and other types of neuropathies. Depending upon the particular indication, the compositions can be targeted to specific areas or tissues of a subject. For example, in some methods, compositions are delivered by injection into the limbs to treat diabetic neuropathies. Other treatments, in contrast, involve administering the composition in a general manner without seeking to target delivery to specific regions.

A number of approaches can be utilized to localize the delivery of agents to particular regions. Certain of these methods involve delivery to the body lumen or to a tissue (see, e.g., U.S. Pat. Nos. 5,941,868; 6,067,988; 6,050,986; and 5,997,509; as well as PCT Publications WO 00/25850; WO 00/04928; 99/59666; and 99/38559). Delivery can also be effectuated by intramuscular or intramyocardial injection or administration. Examples of such approaches include those discussed in U.S. Pat. Nos. 6,086,582; 6,045,565; 6,056,969; and 5,997,525; and in PCT Publications WO 00/16848; WO 00/18462; WO 00/24452; WO 99/49773 and WO 99/49926. Other options for local delivery include intrapericardial injection (see, e.g., U.S. Pat. Nos. 5,931,810; 5,968,010; and 5,972,013) and perivascular delivery. Various transmyocardial revascular (TMR) channel delivery approaches can be utilized as well. Many of these methods utilize a laser to conduct the revascularization. A discussion of such approaches is set forth in U.S. Pat. Nos. 5,925,012; 5,976,164; 5,993,443; and 5,999,678, for example. Other options include intraarterial and/or intracoronary delivery, for example coronary artery injection (see, e.g., WO 99/29251) and endovascular administration (see, e.g., U.S. Pat. Nos. 6,001,350; 6,066,123; and 6,048,332; and PCT Publications WO 99/31982; WO 99/33500; and WO 00/15285).

Additional options for the delivery of compositions to treat neuropathies include systemic administration using intravenous or subcutaneous administration, cardiac chamber access (*see*, *e*.*g*., U.S. Pat. No. 5,924,424) and tissue engineering (U.S. Pat. No. 5,944,754).

Other delivery methods known by those skilled in the art include the methods disclosed in U.S. Pat. Nos. 5,698,531; 5,893,839; 5,797,870; 5,693,622; 5,674,722; 5,328,470; and 5,707,969.

### X. APPLICATIONS

### A. GENERAL

ZFPs that bind to target sites in a VEGF gene (and in other genes), and nucleic acids encoding them, can be utilized to treat a wide variety of neuropathies and neurodegenerative conditions. Such methods generally involve contacting a target site of a nucleic acid within a cell or population of cells with a ZFP that has been engineered to recognize and bind to the target site. Methods can be performed *in vitro* with cell cultures, for example, or *in vivo*. Certain methods are performed such that neuropathies are treated by activating one or more VEGF genes.

### B. THERAPEUTIC APPLICATIONS

The ZFPs provided herein and the nucleic acids encoding them such as in the pharmaceutical compositions described herein can be utilized to modulate (*e*.*g*., activate or repress) expression of genes involved in ameliorating or eliminating neuropathy and/or neurodegeneration. For example, VEGF genes can be activated such that the resulting VEGF proteins can act as neurotrophic factors, thereby facilitating nerve function and/or nerve growth and/or preventing nerve degeneration, both in cell cultures *(i*.*e*., in *in vitro* applications) and *in vivo*.

Hence, certain methods for treating various types of neuropathy (including diabetic neuropathy) and neurodegenerative conditionsinvolve introducing a ZFP into a subject. Binding of the ZFP bearing an activation domain to a gene whose expression ameliorates neuropathy can be used to treat the neuropathy. Certain methods involve the use of ZFPs such as described herein to bind to target sites in VEGF genes. An activation domain fused to the ZFP activates the expression of one or more VEGF genes.

A variety of assays for assessing neurotrophy as it relates to neuropathy (*e*.*g*., nerve function) are known. For example, endothelial cell proliferation assays are discussed by Ferrara and Henzel (1989) Nature 380:439-443; Gospodarowicz et al. (1989) Proc. Natl. Acad. Sci. USA, 86: 7311-7315; and Claffey et al. (1995) Biochim. Biophys. Acta. 1246:1-9. The ability of the ZFPs and/or nucleic acids to promote neurotrophy can be evaluated, for example, in chick chorioallantoic membrane, as discussed by Leung et al. (1989) Science 246:1306-1309. Another option is to conduct assays with rat corneas, as discussed by Rastinejad et al. (1989) Cell 56:345-355. Other assays are disclosed in U.S. Pat. No. 5,840,693. Assays for nerve regeneration include assessment of nerve-endplate contact following crush injury of the recurrent laryngeal nerve in rats. Rubin et al. (2001) Laryngoscope 111:2041-2045; Rubin et al. (2003) Laryngoscope 113:985-989. In addition, microscopic examination of tissue sections can be used to evaluate nerve condition. Nerve blood flow may also be assayed, for example by laser Doppler imaging or direct detection of a locally administered fluorescent lectin analogue, as described for example in Schratzberger (2001) J Clin Invest. 107(9):1083-92. In addition, motor and/or sensory nerve conduction velocities can be assayed, as described in Schratzberger, *supra*. Each of these methods are accepted assays and the results can also be extrapolated to other systems.

The compositions provided herein can also be utilized to repress expression of genes in a variety of therapeutic applications. For example, genes whose products serve to limit nerve growth under normal circumstances can be repressed so as to stimulate nerve growth in various neuropathic conditions such as diabetic neuropathy.

The following examples are provided solely to illustrate in greater detail particular aspects of the disclosed methods and compositions and should not be construed to be limiting in any way.

### EXAMPLE 1: TREATMENT OF PERIPHERAL NEUROPATHY WITH DESIGNED ZINC FINGER PROTEINS

The therapeutic potential of a zinc finger protein designed to recognize a target site within a VEGF gene and activate expression of VEGF was assessed in streptozotocin-treated rats, a validated experimental model of diabetic neuropathy. In this model, diabetes is induced in rat after an overnight fasting by a single intraperitoneal injection of streptozotocin. Streptozotocin (STZ) treatment causes partial destruction of pancreatic β-cells and diabetes was induced within a week. Severe peripheral neuropathy develops in the model and is characterized by a significant slowing of motor and sensory nerve conduction velocities.

A 3-fingered zinc finger protein that recognizes a target site in VEGF-A was designed as described above. The designed ZFP, designated VOP32E, binds to the target site GGGGGTGAC and includes the following amino acid sequences in the recognition helix of each finger: DRSNLTR (finger 1 or F1); TSGHLSR (finger 2 or F2); and RSDHLSR (finger 3 or F3). *See*, *also*, Table 1. A p65 transcriptional activation domain was fused to the VOP32E ZFP.

Diabetes was induced in male Wistar rats after an overnight fast by a single intraperitoneal injection of streptozotocin. Age and weight matched untreated rats were used as controls. Serum glucose level was measured one week after streptozotocin treatment and STZ-treated animals that were identified as diabetic were randomized into 3 treatment groups (n=12).

Treatments were initiated 30 days after the induction of diabetes. A plasmid encoding the VOP32E-p65 was provided in a single dose of three injections into the gastrocnemius and soleus muscle groups of the right leg, at doses of 125 or 500 µg. Similar injections with an empty vector plasmid, VAX-1, served as controls. VOP32E and pVAX-1 plasmids were formulated in 1% poloxamer 407, 150 mM NaCl, 2 mM Tris, pH 8.0.

The contralateral limb served as the uninjected control. Nerve conduction velocities (MNCV and SNCV) were measured in the treated and untreated contralateral limb four weeks after gene delivery, essentially as described in Schratzberger et al., *supra*. Results are shown in Table 4:

| Table. 4 | | | | | |
|---|---|---|---|---|---|
| Motor and sensory nerve conduction velocities in VOP32E, pVAX-1-treated and control groups | | | | | |
| Group | Treatment | Dose (µg) | Final Body Weight (g) | Sciatic Nerve MNCV (m/s) | Sciatic-nerve SNCV (m/s) |
| Control-nondiabetic | none | 0 | 507.7±33.6 | 57.1^{A}±5.5 | 59.3^{A}±9.93 |
| Diabetic | pVAX-1 | 500 | 351.0±45.1 | 48.5^{B}±5.9 | 50.3^{B}±9.33 |
| Diabetic | SGMO-001 | 125 | 351.5±15.23 | 47.2^{C}±10.2 | 59.4^{C}±8.77 |
| Diabetic | SGMO-001 | 500 | 340.8±15.23 | 48.3^{D}±9.4 | 47.2^{D}±7.5 |
| Statistical significance | | | | | p<0.0039 (C vs. B) |

Following SNCV and MNCV measurements, the animals were sacrificed and the injected muscles were dissected and harvested for isolation of RNA. RNA preparations were subsequently analyzed for 32Ep65 transgene expression by real-time PCR.

Delivery of VOP32E to skeletal muscles of diabetic rats led to an increase in the conduction velocity of sensory nerves without affecting motor conduction velocity (Fig. 1). SNCV improvement in the low dose VOP32E (125 µg) treatment group was statistically significantly when compared to pVAX-1 (empty vector) treated animals (p<0.005). SNCV in the low dose treatment group was restored to normal levels within four week after gene transfer (Table 1, Fig. 1). A trend for improved SNCV was observed in the low dose group when compared to the uninjected contralateral limb (p=0.054). Others have also reported an improvement in SNCV without an effect on MNCV (Sayers, NM et al. Diabetes (2003) 52, 2372-2380).

Thus, engineered ZFP VOP32E is capable of significantly improving sensory nerve conduction velocities in an animal model of diabetic neuropathy.

### EXAMPLE 2: DOSE-RESPONSE

To evaluate dose response of VOP32E in the model of peripheral diabetic neuropathy described above, three additional VOP32E treatment groups were added to this study and received doses that were 4-fold lower and 2-fold higher than the effective dose of 125 µg (Example 1). Diabetes was induced as described above. Age and weight matched rats were used as controls. Serum glucose level was measured one week later and animals that were diabetic were then randomized into 3 treatment groups (n=12).

Treatments were initiated 27 days after the induction of diabetes. Animals were given VOP32E at doses of 31.25, 62.5, 125 or 250 µg in the left gastrocnemius muscle. The contralateral limb served as the uninjected control. VOP32E or pVAX-1 plasmid DNA was formulated in 5% poloxamer 188, 150 mM NaCl, and 2 mM Tris pH 8.0. SNCV and MNCV were measured four weeks following gene delivery. Measurements of nerve conduction velocities (MNCV and SNCV) were performed as described above. RNA was processed and analyzed for transgene expression as described above.

In this study, the MNCV and SNCV values showed clear efficacy for the three higher doses of the VOP32E-p65 plasmid.

### EXAMPLE 3: CONDITIONED MEDIUM

Human HEK 293 cells in which VEGF expression was activated using a ZFP activator targeted to the VEGF gene were used as a source of conditioned medium. Such conditioned medium was able to rescue the serum-dependence of two human (SK-N-MC and SHEP-1) and a rat (ND8) neuroblastoma cell lines.

### EXAMPLE 4: CONSTRUCTION OF AN ADENOVIRAL VECTOR ENCODING A VEGF-TARGETED ZINC FINGER PROTEIN TRANSCRIPTION FACTOR

This example describes the construction of a recombinant adenoviral vector, Ad-VOP32Ep65Flag, containing sequences encoding a fusion protein containing a nuclear localization sequence (NLS), the VEGF-targeted VOP32E zinc finger DNA-binding domain (Table 1), a p65 transcriptional activation domain and a Flag epitope tag.

Sequences encoding the fusion protein were assembled as described, for example, in co-owned U.S. Patents 6,453,242 and 6,534,261 and cloned between the *Eco* RI and *Xho* I sites of the pcDNA3 vector (Invitrogen, Carlsbad, CA). The resultant plasmid was digested with *Xho* I, followed by treatment with the DNA Polymerase I Klenow fragment in the presence of dNTPs to convert the *Xho* I ends to blunt ends. This DNA was then digested with *Afl* II (site present in the insert upstream of sequences encoding NLS) and the smaller of the two fragments was purified using a Qiagen gel extraction kit (Qiagen, Valencia, CA).

A DNA fragment containing the human cytomegalovirus immediate early promoter/enhancer (CMV) and two tetracycline operator sequences (TetO₂) was obtained by digesting the pcDNA4/TO plasmid (Invitrogen, Carlsbad, CA) with *Mlu*I and *Afl*II and purifying the smaller of the two fragments using a Qiagen gel extraction kit (Qiagen, Valencia, CA).

The plasmid pShuttle (Clontech, Palo Alto, CA) was digested with *Xba* I, followed by treatment with the DNA Polymerase I Klenow fragment in the presence of dNTPs to convert the *Xba* I ends to blunt ends. The resulting DNA fragment was digested with *Mlu* I, and the larger of the two fragments was purified using a Qiagen gel extraction kit (Qiagen, Valencia, CA).

A three-way ligation was performed, using the *Mlu* I/*Afl* II fragment containing the tetracycline-inducible CMV promoter/enhancer, the *Afl* II/*Xho* I fragment encoding the fusion protein, and the *Xba* I/*Mlu* I fragment of pShuttle (containing a bovine growth hormone polyadenlyation signal and a kanamycin resistance marker) to generate a plasmid having a pShuttle vector backbone containing a transcription unit encoding the fusion protein under the transcriptional control of the tetracycline-inducible CMV promoter/enhancer and the bovine growth hormone polyadenylation signal.

The resulting pShuttle-based plasmid was digested with I-*Ceu* I and PI-*Sce* I. The digestion mixture was extracted with phenol:chloroform:isoamyl alcohol (25:24:1) and precipitated with 0.3M NaOAc/ethanol. The precipitated DNA was resuspended in TE buffer and ligated to 1-*Ceu* I and PI-*Sce* I double-digested pAdeno-X vector (Clontech, Palo Alto, CA). Clones containing an inserted I-*Ceu* I/PI-*Sce* I fragment encoding the fusion protein into the I-*Ceu* I and PI-*Sce* I sites of pAdeno-X were selected.

The recombinant adenoviral vector was packaged into virions following transfection into T-REx^{™}-293 cells (Invitrogen), and adenoviruses were harvested from transfected T-REx^{™}-293 cells that had been lysed with three consecutive freeze-thaw cycles. Recombinant adenoviruses were further amplified in T-REx^{™}-293 cells and purified by two rounds of cesium chloride gradient centrifugation. Purified recombinant adenoviruses (AdVOP32Ep65) were dialyzed against three changes of 10 mM Tris pH8.0, 2 mM MgCl₂, 4% sucrose, and stored in aliquots at - 80°C. Adenoviral particle numbers were determined by absorbance at 260 nm and infectious titers were determined using the Adeno-X Rapid Titer Kit (Clontech, Palo Alto, CA).

### EXAMPLE 5: NEURAL REGENERATION MEDIATED BY A VEGF-TARGETED ZFP TRANSCRIPTIONAL ACTIVATOR FOLLOWING NERVE CRUSH INJURY

The ability of the VOP32E transcriptional activator to enhance neural regeneration was tested in a rat crushed recurrent laryngeal nerve model system. Rubin et al. (2003) The Laryngoscope 113:985-989. It had previously been shown that delivery of an empty adenoviral vector (*i*.*e*., a vector lacking an inserted transgene) to the crushed recurrent laryngeal nerve (RLN) did not cause significant additional neuronal injury. Rubin *et al*. (2003) *supra.*

For this experiment, rats were randomly assigned to 2 groups. In Group I, the RLN was crushed and the nerve was injected with an empty adenoviral vector (control, n= 10). In Group II, the nerve was crushed and injected with AdVOP32Ep65 (n = 10). Regeneration was assayed by counting the number of nerve-endplate contacts in laryngeal muscles. Five rats from each group were sacrificed at 3 d and the remaining 5 rats from each group at 7 d. Laryngeal cryosections were processed for acetylcholine histochemistry (to identify motor endplates,) followed by neurofilament immunoperoxidase (to identify nerve fibers). Percentage nerve-endplate contact, a well-established indicator of nerve regeneration, was determined and compared between groups at 3 d (C) and 7 d (D) according to published protocols. Rubin et al. (2001) Laryngoscope 111:2041-2045; Rubin *et al.* (2003), *supra.*

Results of these analyses indicate that, at 3 days post-crush of the RLN, there was no significant increase in percentage nerve-endplate contact between control- and Adp65-injected animals. However by 7 d, there was a substantial increase in nerve-endplate contact in animals that had been injected with Adp65, compared to animals that had been injected with the empty vector. Histological studies confirmed previous reports (Rubin *et al*., *supra*) that gene delivery of VEGF to neurons does not produce aberrant blood vessel formation. These results show that delivery of an adenovirus vector encoding a VEGF-targeted ZFP transcriptional activator to damaged neural tissue stimulates neural regeneration.

### Example 6: Construction and production of an AAV delivery vehicle encoding a VEGF-targeted zinc finger protein (AAV-VOP32Ep65Flag)

Recombinant AAV vector, pAAV-VOP32Ep65Flag, was created as follows: 1) Plasmid pcDNA3-VOP32Ep65Flag was digested with Pme I and Xho I. The fragment that contains a nuclear localization sequence (NLS), the VOP32E zinc finger DNA-binding domain, a p65 transcriptional activation domain and a flag epitope tag was purified using a Qiagen gel extraction kit. 2) Plasmid pAAV-MCS (Stratagene) was digested with Hinc II and Xho I. The larger fragment from the digestion was purified using a Qiagen gel extraction kit. 3) Fragments from 1) and 2) were ligated to generate the plasmid, pAAV-VOP32Ep65Flag.

pAAV-VOP32Ep65Flag, pAAV-RC (Stratagene) and pHelper (Stratagene) were co-transfected into HEK293 cells (ATCC) by calcium phosphate, and recombinant AAV was harvested from transfected HEK293 cells lysed with two consecutive freeze-thaw cycles. Recombinant AAV was purified using a heparin-agarose suspension (Sigma) and concentrated using Amicon Ultra-15 and Ultra-4 centrifugal filters (Millipore). Purified and concentrated recombinant AAV was dialyzed against three changes of 10 mM Tris pH7.5-115 mM NaCl-1 mM MgCl₂, and stored in aliquots at -80°C. AAV vector genome titer was determined by Taqman.

### Example 7: Treatment of diabetic neuropathy by intramuscular injection of plasmid DNA encoding a VEGF-targeted zinc finger protein

### Formulation

pV-32Ep65 is a plasmid encoding a fusion protein containing, in N- to C-terminal order, a nuclear localization signal, the VOP32E zinc finger binding domain targeted to the VEGF gene and a p65 transcriptional activation domain (Figure 4).

It is formulated as a sterile, injectable solution for intramuscular administration in 3 ml vials(Table 5). The formulation consists of pV-32Ep65 plasmid DNA (2 mg/mL), Poloxamer188 (5% w/v), NaCl (150 mM), 2 mM Tris-HCl, pH 8.0, and sterile water for injection.

**Table 5. Quantitative Composition of injection formulation**

| **Component** | **Unit Formula (mg/mL)** |
|---|---|
| pV-32Ep65 (plasmid DNA) | 2mg |
| Sodium chloride, USP (150 mM NaCL) | 8.76 mg |
| P188, NF (5% w/v) | 50 mg |
| Tromethamine, pH 8.0, USP (2 mM Tris-HCl) | 0.242 mg |
| Sterile Water for Injection, USP | q.s. to 1.0 mL |

The final drug product is tested for appearance, plasmid identity and concentration, poloxamer identity and concentration, moisture content, pH, conductivity, endotoxin, and sterility. Normal saline (0.9% NaCl) is provided in 3 ml vials to serve as the placebo. The formulation is stored at -20°C. Immediately before use, the vial is permitted to equilibrate at room temperature for 10 min. Once thawed, vials will not be reused on subsequent days.

### Administration

Dosing involves both lower limbs, starting distally and moving proximally. In a blinded fashion, one leg is dosed with pV-32Ep65 formulation and the other with placebo. Doses are as follows: Cohort 1: 1 mg, Cohort 2: 5 mg, Cohort 3: 15 mg, Cohort 4: 30 mg, and Cohort 5: 60 mg. The drug is supplied in 3 mL vials and is injected in either 0.5, 1, or 1.5 mL volumes in the foot, calf, and thigh, respectively. Only one injection will be given in the foot and will be injected at the dorsum of the foot into the extensor digitorum brevis. Up to ten 1 mL injections may be given in the lower leg into muscle near target nerve sites for the sural, peroneal, and tibial nerves. Any remaining injections are given in the thigh in 1.5 mL aliquots (up to 13 injections in the anterior and posterior thigh at the maximum dose of 60 mg).

### Injection procedure

The injection site in the muscle allows the deposit of between 1 and 3 individual 0.5 mL doses of the formulation by inserting a needle along the long axis of the muscle fiber (*i*.*e*., parallel with the femoral artery) up to its hub (the needle's full insertion point) and depositing 0.5 mL doses at ½ inch intervals as the needle is withdrawn. The needle is pointed toward the foot and inserted at an approximate 30° angle to the surface of the skin, except at the foot. In the foot, the needle should be angled toward the toes at a slight angle.

### Injection Needles and volumes for each injection site

Needles (25 gauge) with 3 cc syringes are to be used as follows: 1 inch needle for the foot, 1½ inch needle for the calf, and 3 inch needle for the thigh. Injection sites are described below. Care should be taken to avoid injection into any area of skin ulceration.

### Injection site description for each dose level

For each leg, subjects in Cohort 1 receive one IM injection of 0.5 mL (1.0 mg of plasmid) or placebo. The needle is angled toward the toes, and the injection is given at the dorsum of the foot into the extensor digitorum brevis (superficial peroneal nerve).

For each leg, subjects in Cohort 2 receive a total of three IM injections or placebo. One 0.5 mL (1 mg plasmid) injection is given in the foot as described above. Two additional 1 mL injections (2.0 mg plasmid per injection) are placed in the lower leg. One injection is placed into the muscles of the medial lower calf (medial portion of the soleus, flexor digitorum longus near the tarsal tunnel [tibial nerve]), and another is placed into the lateral gastrocnemius just proximal to the achilles tendon (sural nerve).

For each leg, subjects in Cohort 3 receive a total of eight IM injections. One 0.5 mL injection (1.0 mg plasmid or placebo) is given in the foot as described for Cohort 1. Seven additional 1 mL IM injections (2.0 mg plasmid each or placebo) is given in the lower leg. One injection is placed near the tarsal tunnel (tibial nerve), and the second injection is placed into the lateral gastrocnemius just proximal to the achilles tendon (sural nerve) as described for Cohort 2. One 1 mL injection is given at the lateral gastrocnemius muscle near the fibular head (peroneal nerve), and another 1 mL injection is given through the leg into the soleus muscle just inferior to the popliteal fossa (tibial nerve). One additional 1 mL injection is given into the medial portion of the gastrocnemius muscle approximately 5 cm proximal to the achilles tendon. Another 1 mL IM injection is given into the center portion of the tibialis anterior.

For each leg, subjects in Cohort 4 receive a total of 14 IM injections of plasmid or placebo. Injections into the foot and lower leg are given as described for Cohort 3. Three additional 1 mL IM injections are given into the lower leg: one additional 1 mL IM injection is given in the upper portion of the tibialis anterior, and two 1 mL IM injections are given in the medial gastrocnemius (the first of these is given 5 cm superior to the medial gastrocnemius injection just above the achilles tendon described for Cohort 3, and a second IM injection is given 5 cm further superior). Three 1.5 mL IM injections (3 mg of plasmid each or placebo) are given in the thigh. The first of these injections is given into the medial distal-most portion of the medial thigh into the vastus medialis. The second of these injections is given into the lateral distal-most portion of the thigh into the vastus lateralis. The third of these injections is delivered into the quadriceps femoris just proximal to the tendon of the rectus femoris.

For each leg, subjects in Cohort 5 receive a total of 24 IM injections of plasmid or placebo. Injections into the foot, lower leg, and distal thigh are given as described for subjects in Cohort 4. Ten additional 1.5 mL IM injections (3.0 mg of plasmid each or placebo) are given in two rings of five into the thigh. Each ring of injections is placed in a horizontal line approximately 5 cm from each other. The most inferior ring is placed approximately 5 cm superior to the quadriceps femoris injection described for Cohort 4. Injections within each ring are approximately 5 cm apart.

### Clinical Evaluation

Subjects will be assessed for the extent of Diabetic Peripheral Neuropathy by using the following modalities:

### NSS

The NSS includes 17 separate items relating to symptoms of DN. Eight items focus on muscle weakness, five focus on sensory disturbances, and 4 focus on autonomic symptoms. Each item is scored on a binary scale (0 = absent, 1 = present), with the maximal score being 17.

### NIS-LL

The NIS-LL grades muscle strength, sensory modalities, and deep tendon reflexes of the lower extremities. Both legs will be assessed. Muscle strength is graded on a scale of 0-4. Sensory modalities and reflexes are scored on a scale of 0 to 2. A maximum score of 64 is possible.

### TNS

The TNS assesses symptoms, signs, QST for vibration, and nerve conduction attributes of DN. Ten modalities are assessed, and each is scored on a scale of 0-4. A maximum score of 40 is possible.

### Nerve Conduction Studies

Standard NCS of bilateral peroneal nerves, sural nerves, and tibial nerves will be performed on all subjects.

### Quantitative Sensory Testing (QST)

QST, including measurement ofVPTs, will be measured at the great toes, bilaterally, by using a CASE IV instrument (WR Medical Electronics, Stillwater, MN).

### Skin Biopsies

Skin biopsies will be done on the bilateral lower legs 10 cm proximal to the lateral malleolus at Days 0 and 180to determine the density of intraepidermal nerve fibers.

## Claims

1. Use of a nucleic acid, wherein the nucleic acid encodes a polypeptide, wherein the polypeptide comprises:
(i) a zinc finger DNA-binding domain that is engineered to bind to a target site in the vascular endothelial growth factor-A (VEGF-A) gene; and
(ii) a transcriptional activation domain;
wherein the nucleic acid can be expressed in one or more cells of a subject to be treated, whereby the polypeptide is capable of binding to the target site and activating transcription of the VEGF-A gene for the manufacture of a composition for treating a neuropathic or neurodegenerative condition in a subject.

2. The use according to claim 1, wherein the zinc finger DNA-binding domain comprises three zinc fingers and the amino acid sequence of the recognition regions of the zinc fingers is as follows:
F1: DRSNLTR (SEQ ID NO:55)
F2: TSGHLSR (SEQ ID NO:141)
F3: RSDHLSR (SEQ ID NO:68).

3. The use according to claim 1 or 2, wherein the transcriptional activation domain is a p65 activation domain.

4. Use of a polypeptide, wherein the polypeptide comprises:
(i) a zinc finger DNA-binding domain that is engineered to bind to a target site in the vascular endothelial growth factor-A (VEGF-A) gene; and
(ii) transcriptional activation domain;
whereby the polypeptide is capable of binding to the target site and activating transcription of the VEGF-A gene for the manufacture of a composition for treating a neuropathic or neurodegenerative condition in a subject.

5. The use according to claim 4, wherein the zinc finger DNA-binding domain comprises three zinc fingers and the amino acid sequence of the recognition regions of the zinc fingers is as follows:
F1: DRSNLTR (SEQ ID NO:55)
F2: TSGHLSR (SEQ ID NO:141)
F3: RSDHLSR (SEQ ID NO:68).

6. The use according to claim 4 or 5, wherein the transcriptional activation domain is a p65 activation domain.

## Patentansprüche

1. Verwendung einer Nukleinsäure, wobei die Nukleinsäure ein Polypeptid kodiert, wobei das Polypeptid umfasst:
(i) eine Zinkfinger-DNA-Bindungsdomäne, die so konzipiert ist, dass sie an eine Zielstelle in dem Gen des vaskulären endothelialen Wachstumsfaktors A (VEGF-A) bindet; und
(ii) eine Transkriptionsaktivierungsdomäne;
wobei die Nukleinsäure in ein oder mehreren Zellen eines zu behandelnden Subjekts exprimiert werden kann, wobei das Polypeptid in der Lage ist, an die Zielstelle zu binden und die Transkription des VEGF-A Gens zu aktivieren, zur Herstellung einer Zusammensetzung zur Behandlung eines neuropathischen oder neurodegenerativen Zustandes bei einem Subjekt.

2. Verwendung nach Anspruch 1, wobei die Zinkfinger-DNA-Bindungsdomäne drei Zinkfinger umfasst und die Aminosäuresequenz der Erkennungsbereiche der Zinkfinger wie folgt ist;
F1: DRSNLTR (SEQ ID NO: 55)
F2: TSGHLSR (SEQ ID NO: 141)
F3: RSDHLSR (SEQ ID NO: 68).

3. Verwendung nach Anspruch 1 oder 2, wobei die Transkriptionsaktivierungsdomäne eine p65-Aktivierungsdomäne ist.

4. Verwendung eines Polypeptids, wobei das Polypeptid umfasst:
(i) eine Zinkfinger-DNA-Bindungsdomäne, die so konzipiert ist, dass sie an eine Zielstelle in dem Gen des vaskulären endothelialen Wachstumsfaktors A (VEGF-A) bindet; und
(ii) eine Transkriptionsaktivierungsdomäne;
wobei das Polypeptid in der Lage ist, an die Zielstelle zu binden und die Transkription des VEGF-A Gens zu aktivieren,
zur Herstellung einer Zusammensetzung zur Behandlung eines neuropathischen oder neurodegenerativen Zustandes bei einem Subjekt.

5. Verwendung nach Anspruch 4, wobei die Zinkfinger-DNA-Bindungsdomäne drei Zinkfinger umfasst und die Aminosäuresequenz der Erkennungsbereiche der Zinkfinger wie folgt ist;
F1: DRSNLTR (SEQ ID NO: 55)
F2: TSGHLSR (SEQ ID NO: 141)
F3: RSDHLSR (SEQ ID NO: 68).

6. Verwendung nach Anspruch 4 oder 5, wobei die Transkriptionsaktivierungsdomäne eine p65-Aktivierungsdomäne ist.

## Revendications

1. Utilisation d'un acide nucléique, ledit acide nucléique codant pour un polypeptide, ledit polypeptide comprenant :
(i) un domaine de liaison de l'ADN en doigt de zinc qui est modifié pour se lier à un site cible dans le gène du facteur de croissance de l'endothélium vasculaire A (VEGF-A) ; et
(ii) un domaine d'activation de la transcription ;
ledit acide nucléique pouvant être exprimé dans une ou plusieurs cellules d'un sujet à traiter, de sorte que le polypeptide soit capable de se lier au site cible et d'activer la transcription du gène du VEGF-A, pour la fabrication d'une composition destinée au traitement d'une affection neuropathique ou neurodégénérative chez un sujet.

2. Utilisation selon la revendication 1, ledit domaine de liaison de l'ADN en doigt de zinc comprenant trois doigts de zinc et la séquence d'acides aminés des régions de reconnaissance des doigts de zinc étant la suivante :
F1 : DRSNLTR (SEQ ID NO : 55)
F2 : TSGHLSR (SEQ ID NO : 141)
F3 : RSDHLSR (SEQ ID NO : 68).

3. Utilisation selon la revendication 1 ou 2, ledit domaine d'activation de la transcription étant un domaine d'activation de p65.

4. Utilisation d'un polypeptide, ledit polypeptide comprenant :
(i) un domaine de liaison de l'ADN en doigt de zinc qui est modifié pour se lier à un site cible dans le gène du facteur de croissance de l'endothélium vasculaire A (VEGF-A) ; et
(ii) un domaine d'activation de la transcription ;
de sorte que le polypeptide est capable de se lier au site cible et d'activer la transcription du gène du VEGF-A, pour la fabrication d'une composition destinée au traitement d'une affection neuropathique ou neurodégénérative chez un sujet.

5. Utilisation selon la revendication 4, ledit domaine de liaison de l'ADN en doigt de zinc comprenant trois doigts de zinc et la séquence d'acides aminés des régions de reconnaissance des doigts de zinc étant la suivante :
F1 : DRSNLTR (SEQ ID NO : 55)
F2 : TSGHLSR (SEQ ID NO : 141)
F3 : RSDHLSR (SEQ ID NO : 68).

6. Utilisation selon la revendication 4 ou 5, ledit domaine d'activation de la transcription étant un domaine d'activation de p65.
